# EUROPEAN PATENT APPLICATION

(11) **EP 3 838 925 A1**
(43) Date of publication of application: **23.06.2021**
(21) Application number: 19857371.9
(22) Date of filing: 04.09.2019
(51) Int. Cl.: C07K 19/00, C12N 15/62, A61K 38/26, A61K 47/68, A61P 3/10, A61P 3/04

(54) **LONG-ACTING RECOMBINANT GLP1-FC-CD47 PROTEIN, PREPARATION METHOD AND USE THEREOF**

(30) Priority: 06.09.2018 CN 201811039238
(71) Applicant: Zhejiang Palo Alto Pharmaceuticals, Inc., Quzhou, Zhejiang 324099 (CN)
(72) Inventor: XI, Zhijian, Quzhou, Zhejiang 324099 (CN); XU, Huaqiang, Quzhou, Zhejiang 324099 (CN); WANG, Gaihong, Quzhou, Zhejiang 324099 (CN)
(74) Representative: Dargiewicz, Joanna
(86) International application number: PCT/CN2019/104439
(87) International publication number: WO 2020/048494

(57) **Abstract**

The present invention relates to a long-acting recombinant GLP-1-Fc fusion protein containing CD47 signals and preparation process as well as use thereof. To be specific, the present invention relates to a novel recombinant fusion protein which includes GLP-1 polypeptides, Fc fragments and CD47 signal polypeptides. This fusion protein has been found for the first time to be able to exist as a tetramer, more stable than the homodimeric Fc fusion protein, and with effectively prolonged half-life in vivo. The present invention also mutates GLP-1 and Fc fragments to enhance GLP-1 biological activities and reduce antibody-dependent cell-mediated cytotoxicity (ADCC) effects so as to decrease potential cytotoxicities and immunogenicity. Furthermore, the initial combination of the CD47 signal polypeptide with the fusion protein in this fusion protein can reduce macrophage clearance of the polypeptide and decrease dosing frequency in humans.

## Description

### Technical Field

The present invention is related to expertise in medical field, specifically involving a long-acting recombinant GLP-1-Fc fusion protein containing CD47 signals and preparation and use thereof.

### Background Art

A glucagon-like peptide-1 (GLP-1) is an incretin secreted by intestinal L cells, and it exerts physiological effects by acting on the GLP-1 receptor (GLP-1R). This receptor, which belongs to the G protein-coupled receptor family, activates cyclic adenosine monophosphate (cAMP) via G proteins to elevate intracellular cAMP levels and activates protein kinase A to lead to the intracellular cascades. Under normal physiological conditions, the main physiological functions of GLP-1 are to stimulate insulin secretion by pancreatic β cells, inhibit glucagon secretion by pancreatic α cells, restrain appetite, and delay gastric emptying; more importantly, the insulinotropic effect of GLP-1 is dependent on blood glucose level. For patients with non insulin-dependent diabetes (type 2 diabetes mellitus), the insulinotropic and glucagon-inhibiting functions of GLP-1 are impaired, but not completely abolished, and can be reversed by physiological or supraphysiological GLP-1 concentrations. Continuous administration of GLP-1 in patients with type 2 diabetes can improve the glycemic control and reduce the body weight. Therefore, GLP-1 represents an ideal therapeutic target for the treatment of type 2 diabetes.

However, native GLP-1 has a very short in vivo half-life (about 2-3 minutes), as it can be degraded mainly by dipeptidyl peptidase IV (DPPIV) enzymes in the blood after it enters into blood circulation, and as a result, the clinical application of native GLP-1 is limited. Two major strategic solutions include the development of GLP-1 receptor agonists with longer half-lives or inhibitors of DPPIV enzymes, both of which are currently available in the market. The GLP-1-based drug therapy has the main advantage of lower risk of hypoglycemia compared with other diabetes therapies; GLP-1 receptor agonists can obtain additional beneficial effects of weight loss and protection of beta-cell function. GLP-1 receptor agonists have shown good antihyperglycemic efficacy in clinical settings, with the ability to decrease HbAlc resembles that of thiazolidinediones, low-dose dimethylbipurines and sulfonamides, and outshines α-glucosidase inhibitors and DPPIV inhibitors. For GLP-1 receptor agonists, its common adverse reactions are mainly gastrointestinal reactions, such as nausea and vomiting, possibly due to its effect in gastric emptying prolongation. Moreover, long-acting GLP-1 receptor agonist can alleviate gastrointestinal side effects.

GLP-1 receptor agonists currently approved for marketing by FDA include early short-acting agonists such as exenatide (twice a day), liraglutide, lixisenatide (once a day), and recent long-acting agonists (once a week) such as exenatide microsphere dosage forms, albiglutide, dulaglutide, and semaglutide. Dulaglutide can extend the half-life of GLP-1 in blood by fusing the Fc terminal of the human immunoglobulin G4 (IgG4) with GLP-1. In China, exenatide, liraglutide and exenatide microsphere injections are GLP-1 receptor agonists approved for marketing by CFDA. Dulaglutide, as a marketed GLP-1-Fc fusion protein, has a half-life of only 4.5-4.7 days in vivo. Currently, the lowest dosing frequency of all the marketed GLP-1 receptor agonists is once a week.

Therefore, there is an urgent need to develop a novel, safe drug with longer-acting time for the patients with diabetes.

### Summary

The present invention is aimed at providing a novel, safe drug with longer-acting time for patients with diabetes.

In the first aspect, the present invention provides a fusion protein with a structure as shown in Formula I below:

A-B-C-D (I)

in which each "-" represents independently a linker peptide or a peptide bond;
A is absent or a CD47 signal polypeptide;
B is a GLP-1 polypeptide;
C is an Fc fragment;
D is absent or a CD47 signal polypeptide; and
A and D are not absent at the same time.

In another preferred embodiment, the A is absent and the D is a CD47 signal polypeptide.

In another preferred embodiment, the structure of the fusion protein is shown in Formula II below:

B-C-D (II)

in which B, C, and "-" are defined as described above (Formula I), and D is a CD47 signal polypeptide.

In another preferred embodiment, the GLP-1 polypeptide includes a native GLP-1 and a GLP-1 mutant.

In another preferred embodiment, the sequence of the native GLP-1 is shown in SEQ ID NO.: 1.

In another preferred embodiment, the GLP-1 mutant corresponds to a native GLP-1 with one of the amino acid mutations selected from the following group: alanine (Ala) at position 8, glycine (Gly) at position 22, lysine (Lys) at position 34, arginine (Arg) at position 36, or a their combination thereof.

In another preferred embodiment, the GLP-1 mutant corresponds to a native GLP-1 with one of the amino acid mutations including A8G, G22E, K34R, R36G, R36A, R36V, R36L, or their combination.

In another preferred embodiment, the sequence of the GLP-1 mutant is shown as any one in SEQ ID NOs.: 2-7.

In another preferred embodiment, the sequence of the GLP-1 polypeptide is shown in any one in SEQ ID NOs.: 1-7.

In another preferred embodiment, the Fc fragment is one from an immunoglobulin IgG, preferably the Fc fragment of IgG4, while more preferably the combination of a hinge region, CH2 and CH3 domains of the IgG4.

In another preferred embodiment, the Fc fragment contains a native Fc fragment and an Fc mutant.

In another preferred embodiment, the Fc mutant is an Fc fragment of the IgG4 with one of the mutations including S228P, F234A, L235A, or their combination.

In another preferred embodiment, the amino acid sequence of the Fc fragment is shown in SEQ ID NO.: 9.

In another preferred embodiment, the CD47 signal polypeptide is a polypeptide with CD47 abilities or activity.

In another preferred embodiment, the CD47 abilities or activity refers to a function that shields the CD47 signal polypeptide from being phagocytosed by macrophages.

In another preferred embodiment, the CD47 signal polypeptide is a polypeptide with an epitope that specifically binds to SIRPa.

In another preferred embodiment, the CD47 signal polypeptide is a polypeptide containing a core sequence as shown in SEQ ID NO.: 10.

In another preferred embodiment, the CD47 includes CD47 proteins of human, murine, or other species origin.

In another preferred embodiment, the CD47 signal polypeptide includes a full-length protein of CD47, an active fragment of the CD47 protein, a mutant, derivatives, or analogues.

In another preferred embodiment, the active fragment, mutant, derivatives or analogues of the CD47 protein possess CD47 abilities or activity, preferably containing the core sequence GNYTCEVTELTREGETIIELK (SEQ ID NO.: 10).

In another preferred embodiment, the amino acid sequence of the CD47 signal polypeptide is shown in SEQ ID NO.: 10 or 21.

In another preferred embodiment, the B and the C are linked by a linker peptide P1, which is flexible so that GLP-1 in the fusion protein can effectively bind to the downstream GLP-1 receptor.

In another preferred embodiment, the linker peptide P1 consists of glycine and serine, and preferably, the amino acid sequence of the linker peptide P1 is shown in SEQ ID NO.: 8 (Gly-Gly-Gly-Ser-Gly-Gly-Gly-Gly-Ser-Gly-Gly-Gly-Gly-Ser).

In another preferred embodiment, the C and the D are linked by a linker peptide P2 that contains 3-4 glycines, and preferably is composed of 3-4 glycines.

In another preferred embodiment, the fusion protein has an amino acid sequence as shown in SEQ ID NO.: 13, 14 or 15.

In the second aspect, the present invention provides an oligomer that contains the subject fusion protein of the first aspect of this invention.

In another preferred embodiment, the oligomer is a dimer, a trimer, a tetramer, or a pentamer.

In another preferred embodiment, the oligomer is a tetramer of the subject fusion protein of the first aspect of this invention.

In the third aspect, the present invention provides an isolated polynucleotide which encodes the subject fusion protein of the first aspect of this invention.

In another preferred embodiment, the polynucleotide has a sequence as shown in SEQ ID NO.: 18, 19 or 20.

In the fourth aspect, the present invention provides a carrier that contains the polynucleotide of the third aspect of this invention.

In another preferred embodiment, the carrier is one selected from DNA, RNA, plasmids, lentiviral carriers, adenoviral carriers, retroviral carriers, transposons, or their combination.

In another preferred embodiment, the carrier is a plasmid, and preferably baculovirus plasmid (Bacmid DNA).

In the fifth aspect, the present invention provides a host cell that contains the carrier of the fourth aspect of this invention, or integrates the exogenous polynucleotide of the third aspect of this invention in chromosomes, or expresses the fusion protein of the first aspect of this invention, or expresses the oligomer of the second aspect of this invention.

In another preferred embodiment, the host cell is a mammalian cell.

In another preferred embodiment, the host cell is a HEK293 cell.

In the sixth aspect, the present invention provides a pharmaceutical composition that contains the fusion protein of the first aspect or the oligomer of the second aspect of this invention, and pharmaceutically acceptable carriers or excipients.

In another preferred embodiment, the pharmaceutical composition is used for the treatment of diabetes, obesity, and other diseases that can benefit from reduction in blood glucose and/or body weight.

In the seventh aspect, the present invention provides the fusion protein of the first aspect of the present invention, the oligomer of the second aspect of the present invention, the polynucleotide of the third aspect of the present invention, the carrier of the fourth aspect of the present invention and the use of the host cell of the fifth aspect of this invention. And the use includes preparation of a drug or formulation which can prevent and/or treat diabetes, obesity, and other diseases that can benefit from reduction in blood glucose and/or body weight.

In another preferred embodiment, the diabetes is type 2 diabetes or its related diseases.

In the eighth aspect, the present invention provides the fusion protein of the first aspect of the present invention, the oligomer of the second aspect of the present invention, or the use of the pharmaceutical composition of the sixth aspect of the present invention. And the use includes prevention and/or treatment of diabetes, obesity, and other diseases which can benefit from reduction in blood glucose and/or body weight, preferably type 2 diabetes or its related diseases.

In the ninth aspect, the present invention provides a method for treating several diseases, including administering to a subject in need of treatment an appropriate amount of the fusion protein of the first aspect of this invention, the oligomer of the second aspect or the pharmaceutical composition of the sixth aspect of this invention.

In another preferred embodiment, the subject disease is diabetes, obesity, and other diseases that can benefit from reduction in blood glucose and/or body weight, preferably type 2 diabetes or its related diseases.

In the tenth aspect, the present invention provides a method for preparing the fusion protein of the first aspect of this invention. The method includes Step (a): culturing the host cell of the fifth aspect of this invention to obtain the fusion protein of the first aspect of this invention.

In another preferred embodiment, the method also includes Step (b): purifying the fusion protein obtained in Step (a).

In the eleventh aspect, the present invention provides a GLP-1 mutant which is a native GLP-1 with one of the amino acid mutations, including alanine (Ala) at position 8, glycine (Gly) at position 22, lysine (Lys) at position 34 and arginine (Arg) at position 36, or their combination.

In another preferred embodiment, the GLP-1 mutant is a native GLP-1 with one of the mutations, including A8G, G22E, K34R, R36G, R36A, R36V and R36L, or their combination.

In another preferred embodiment, the GLP-1 mutant is selected from the following group:
(a) An amino acid sequence with the following mutant polypeptides of A8G, G22E, K34R and R36G compared with a sequence shown in SEQ ID NO.: I;
(b) An amino acid sequence with the following mutant polypeptides of A8G, G22E, K34R and R36V compared with a sequence shown in SEQ ID NO.: I;
(c) An amino acid sequence with the following mutant polypeptides of A8G, G22E, K34R and R36L compared with a sequence shown in SEQ ID NO.: I;
(d) An amino acid sequence with the following mutant polypeptides of A8G, G22E, K34R and R36A compared with a sequence shown in SEQ ID NO.: I.

In another preferred embodiment, the sequence of the GLP-1 mutant is shown as any one SEQ ID NOs.: 4-7.

It is understood that, within the scope of this invention, the above technical properties of the present invention and the technical properties specifically described below (such as embodiments) can be combined mutually to constitute a new or preferred technical solution. Due to space limitations, further description won't be repeated here.

### Brief Description of Drawings

Fig.1 shows recombinant protein SDS-PAGE and non-reduced SDS-PAGE protein gel figures wherein 1 presents protein marker, 2 L1, 3 L1-mu, 4 L1-self and 5 L1-self-mu.
Fig.2 shows the stability results of L1-self family recombinant proteins in rat serum in vitro wherein the stability of drugs L1, L1-self, L1-self-mu, L1T-mu and L1-mu is provided with the activity at day 0 as 100%, at respective concentrations of 0.03 nM.
Fig.3 shows the activity determination results of recombinant proteins of the L1 family in animals wherein the drug is subcutaneously administrated at 30 nmol/kg; and the activity of the L1 family is significantly higher as compared with the vehicle control group (*p< 0.05, **p< 0.01, and ***p< 0.001).

### Detailed Description of Embodiments

After extensive and in-depth studies, the inventors obtained for the first time a novel, safe and long-acting drug for the treatment of type 2 diabetes. This drug is a long-acting recombinant GLP-1-Fc fusion protein containing GLP-1, an Fc fragment and CD47 signal polypeptide. This fusion protein has been found for the first time to be able to exist as a tetramer, more stable than the homodimeric Fc fusion protein, and with effectively prolonged half-life in vivo. This invention also rendered mutations of GLP-1 and Fc fragments to enhance GLP-1 biological activities and reduce ADCC effects, so as to decrease potential cytotoxicity and immunogenicity. Furthermore, the initial combination of the CD47 signal polypeptide with the fusion protein in this subject fusion protein can reduce macrophage clearance of the polypeptide and is expected to achieve a lower dosing frequency in humans. Based on the above strategies, the inventors have completed the present invention.

### Glucagon-like peptide-1 (GLP-1)

A glucagon-like peptide-1 (GLP-1) is an incretin secreted by intestinal L cells, and exerts physiological effects by acting on the GLP-1 receptor (GLP-1R). GLP-1R, which belongs to the G protein-coupled receptor family, activates cyclic adenosine monophosphate (cAMP) via G proteins to elevate intracellular cAMP levels and activates protein kinase A to lead to the intracellular cascades. Under normal physiological conditions, the main physiological functions of GLP-1 are to stimulate insulin secretion by pancreatic β cells, inhibit glucagon secretion by pancreatic α cells, restrain appetite, and delay gastric emptying; more importantly, the insulinotropic effect of GLP-1 is dependent on blood glucose concentration. For patients with type 2 diabetes, the insulinotropic and glucagon-inhibiting functions of GLP-1 are impaired, but not completely abolished, and can be reversed by physiological or supraphysiological GLP-1 concentrations. Continuous administration of GLP-1 in patients with type 2 diabetes can improve the glycemic control and reduce the body weight. Therefore, GLP-1 represents an ideal drug target for the treatment of type 2 diabetes.

GLP-1 receptor agonists currently approved for marketing by FDA include early short-acting agonists such as exenatide (twice a day), liraglutide, lixisenatide (once a day), and recent long-acting agonists (once a week) such as exenatide microsphere dosage forms, albiglutide, dulaglutide, and semaglutide, wherein dulaglutide can extend the half-life of GLP-1 in blood by fusing the Fc terminal of the human immunoglobulin G4 (IgG4) with GLP-1. IgG4 has a half-life of approximately 21 days in vivo, whereas the marketed GLP-1-Fc fusion protein (dulaglutide) has a half-life of only 4.5-4.7 days in vivo. Dulaglutide is a dimer with a monomeric molecular weight of 29.8 kDa, and can pass through the glomerular filtration membrane due to its small molecule with accelerated clearance.

In the present invention, as described in the first aspect, GLP-1 polypeptide includes a native GLP-1 and a GLP-1 mutant. In another preferred embodiment, the sequence of the native GLP-1 is shown in SEQ ID NO.: 1. In another preferred embodiment, the sequence of the GLP-1 mutant is shown as any one in SEQ ID NOs.: 2-7. 
SEQ ID NO.: 1 GLP1 (7-37) amino acid sequence

7His-8Ala-Glu-Gly-Thr-Phe-Thr-Ser-Asp-Val-Ser-Ser-Tyr-Leu-Glu-Gly-Gln-Ala-Ala-Lys-Glu-Phe-Ile-

Ala-Trp-Leu-Val-Lys-Gly-Arg-Gly
 SEQ ID NO.: 2 Gly8-GLP1 (7-37) amino acid sequence 
⁷His-⁸Gly-Glu-Gly-Thr-Phe-Thr-Ser-Asp-Val-Ser-Ser-Tyr-Leu-Glu-Gly-Gln-Ala-Ala-Lys-Glu-Phe-Ile-
Ala-Trp-Leu-Val-Lys-Gly-Arg-Gly
 
SEQ ID NO.: 3 Gly8-Glu22-Gly36-GLP1 (7-37) amino acid sequence

7His-8Gly-Glu-Gly-Thr-Phe-Thr-Ser-Asp-Val-Ser-Ser-Tyr-Leu-Glu-22Glu-Gln-Ala-Ala-Lys-Glu-Phe-Ile-

Ala-Trp-Leu-Val-Lys-Gly-36Gly-Gly
 
SEQ ID NO.: 4 Gly8-Glu22-Arg34-Gly36-GLP1 (7-37) amino acid sequence

7His-8Gly-Glu-Gly-Thr-Phe-Thr-Ser-Asp-Val-Ser-Ser-Tyr-Leu-Glu-22Glu-Gln-Ala-Ala-Lys-Glu-Phe-Ile-

Ala-Trp-Leu-Val-34Arg--Gly-36Gly-Gly
 
SEQ ID NO.: 5 Gly8-Glu22-Arg34-Val36-GLP1 (7-37) amino acid sequence

⁷His-⁸Gly-Glu-Gly-Thr-Phe-Thr-Ser-Asp-Val-Ser-Ser-Tyr-Leu-Glu-²²Glu-Gln-Ala-Ala-Lys-Glu-Phe-Ile-

Ala-Trp-Leu-Val-³⁴Arg-Gly-³⁶Val-Gly
 SEQ ID NO.: 6 Gly8-Glu22-Arg34-Leu36-GLP1 (7-37) amino acid sequence

SEQ ID NO.: 7 Gly8-Glu22-Arg34-Ala36-GLP1 (7-37) amino acid sequence

7His-8Gly-Glu-Gly-Thr-Phe-Thr-Ser-Asp-Val-Ser-Ser-Tyr-Leu-Glu-22Glu-Gln-Ala-Ala-Lys-Glu-Phe-Ile-

Ala-Trp-Leu-Val-34Arg-Gly-36Ala-Gly

### An Fc fragment

HumanIgGs are the main form of antibodies in human body and can be categorized into four subtypes: IgG1, IgG2, IgG3 and IgG4. The present invention selects the Fc portion of IgG4 to fuse GLP-1 in order to reduce ADCC effects and decrease potential cytotoxicity. The present invention preferably introduces amino acid mutations (F234A and L235A) into the Fc fragment of the IgG4 to further reduce ADCC effects and decrease cytotoxicity. And this invention preferably introduces an amino acid mutation (S228P) into the Fc fragment of the IgG4 to reduce the formation of GLP-1-Fc monomers.

In another preferred embodiment, the amino acid sequence of the Fc fragment is shown in SEQ ID NO.: 9.

Ala-Glu-Ser-Lys-Tyr-Gly-Pro-Pro-Cys-Pro-Pro-Cys-Pro-Ala-Pro-Glu-Ala-Ala-Gly-Gly-Pro-Ser-Val-
Phe-Leu-Phe-Pro-Pro-Lys-Pro-Lys-Asp-Thr-Leu-Met-Ile-Ser-Arg-Thr-Pro-Glu-Val-Thr-Cys-Val-Val-
Val-Asp-Val-Ser-Gln-Glu-Asp-Pro-Glu-Val-Gln-Phe-Asn-Trp-Tyr-Val-Asp-Gly-Val-Glu-Val-His-Asn-

Ala-Lys-Thr-Lys-Pro-Arg-Glu-Glu-Gln-Phe-Asn-Ser-Thr-Tyr-Arg-Val-Val-Ser-Val-Leu-Thr-Val-Leu-
His-Gln-Asp-Trp-Leu-Asn-Gly-Lys-Glu-Tyr-Lys-Cys-Lys-Val-Ser-Asn-Lys-Gly-Leu-Pro-Ser-Ser-Ile-
Glu-Lys-Thr-Ile-Ser-Lys-Ala-Lys-Gly-Gln-Pro-Arg-Glu-Pro-Gln-Val-Tyr-Thr-Leu-Pro-Pro-Ser-Gln-
Glu-Glu-Met-Thr-Lys-Asn-Gln-Val-Ser-Leu-Thr-Cys-Leu-Val-Lys-Gly-Phe-Tyr-Pro-Ser-Asp-Ile-Ala-
Val-Glu-Trp-Glu-Ser-Asn-Gly-Gln-Pro-Glu-Asn-Asn-Tyr-Lys-Thr-Thr-Pro-Pro-Val-Leu-Asp-Ser-Asp-
Gly-Ser-Phe-Phe-Leu-Tyr-Ser-Arg-Leu-Thr-Val-Asp-Lys-Ser-Arg-Trp-Gln-Glu-Gly-Asn-Val-Phe-Ser-
Cys-Ser-Val-Met-His-Glu-Ala-Leu-His-Asn-His-Tyr-Thr-Gln-Lys-Ser-Leu-Ser-Leu-Ser-Leu-Gly (SEQ
ID NO.: 9)

### A CD47 signal polypeptide

In the present invention, the CD47 signal polypeptide is a polypeptide with CD47 abilities or activity.

In another preferred embodiment, the CD47 abilities or activity refers to a function that protects the CD47 signal polypeptide from being phagocytosed by macrophages.

In another preferred embodiment, the CD47 signal polypeptide is a polypeptide with an epitope that specifically binds to SIRPα.

In another preferred embodiment, the CD47 signal polypeptide is a polypeptide containing a core sequence (self) as shown in SEQ ID NO.: 10.

In another preferred embodiment, the CD47 includes CD47 proteins of human, murine, or other species origin.

In another preferred embodiment, the CD47 signal polypeptide includes a full-length protein of CD47, an active fragment of the CD47 protein, a mutant, derivatives, or analogues.

In another preferred embodiment, the active fragment, mutant, derivatives or analogues of the CD47 protein possess CD47 abilities or activity, preferably containing the core sequence (self) GNYTCEVTELTREGETIIELK (SEQ ID NO.: 10).

In another preferred embodiment, the amino acid sequence of the CD47 signal polypeptide is shown in SEQ ID NO.: 10 or 21.

The present invention can develop a more stable tetramer by introducing a CD47 signal polypeptide into the fusion protein so as to reduce glomerular filtration and decrease the macrophage clearance of the polypeptide, thereby significantly prolonging the duration of therapeutic efficacy and reducing the dosing frequency in humans.

In another preferred embodiment, an amino acid sequence of the CD47 signal polypeptide is shown in SEQ ID NO.: 10.

Gly-Asn-Tyr-Thr-Cys-Glu-Val-Thr-Glu-Leu-Thr-Arg-Glu-Gly-Glu-Thr-Ile-Ile-Glu-Leu-Lys (SEQ ID NO.: 10)

In another preferred embodiment, an amino acid sequence of a human CD47 protein is shown in SEQ ID NO.: 21.

Met-Trp-Pro-Leu-Val-Ala-Ala-Leu-Leu-Leu-Gly-Ser-Ala-Cys-Cys-Gly-Ser-Ala-Gln-Leu-Leu-Phe-Asn-
Lys-Thr-Lys-Ser-Val-Glu-Phe-Thr-Phe-Cys-Asn-Asp-Thr-Val-Val-Ile-Pro-Cys-Phe-Val-Thr-Asn-Met-
Glu-Ala-Gln-Asn-Thr-Thr-Glu-Val-Tyr-Val-Lys-Trp-Lys-Phe-Lys-Gly-Arg-Asp-Ile-Tyr-Thr-Phe-Asp-
Gly-Ala-Leu-Asn-Lys-Ser-Thr-Val-Pro-Thr-Asp-Phe-Ser-Ser-Ala-Lys-Ile-Glu-Val-Ser-Gln-Leu-Leu-Lys-
Gly-Asp-Ala-Ser-Leu-Lys-Met-Asp-Lys-Ser-Asp-Ala-Val-Ser-His-Thr-Gly-Asn-Tyr-Thr-Cys-Glu-Val-
Thr-Glu-Leu-Thr-Arg-Glu-Gly-Glu-Thr-Ile-Ile-Glu-Leu-Lys-Tyr-Arg-Val-Val-Ser-Trp-Phe-Ser-Pro-Asn-
Glu-Asn-Ile-Leu-Ile-Val-Ile-Phe-Pro-Ile-Phe-Ala-Ile-Leu-Leu-Phe-Trp-Gly-Gln-Phe-Gly-Ile-Lys-Thr-
Leu-Lys-Tyr-Arg-Ser-Gly-Gly-Met-Asp-Glu-Lys-Thr-Ile-Ala-Leu-Leu-Val-Ala-Gly-Leu-Val-Ile-Thr-Val-
Ile-Val-Ile-Val-Gly-Ala-Ile-Leu-Phe-Val-Pro-Gly-Glu-Tyr-Ser-Leu-Lys-Asn-Ala-Thr-Gly-Leu-Gly-Leu-
Ile-Val-Thr-Ser-Thr-Gly-Ile-Leu-Ile-Leu-Leu-His-Tyr-Tyr-Val-Phe-Ser-Thr-Ala-Ile-Gly-Leu-Thr-Ser-
Phe-Val-Ile-Ala-Ile-Leu-Val-Ile-Gln-Val-Ile-Ala-Tyr-Ile-Leu-Ala-Val-Val-Gly-Leu-Ser-Leu-Cys-Ile-Ala-
Ala-Cys-Ile-Pro-Met-His-Gly-Pro-Leu-Leu-Ile-Ser-Gly-Leu-Ser-Ile-Leu-Ala-Leu-Ala-Gln-Leu-Leu-Gly-
Leu-Val-Tyr-Met-Lys-Phe-Val-Ala-Ser-Asn-Gln-Lys-Thr-Ile-Gln-Pro-Pro-Arg-Lys-Ala-Val-Glu-Glu-Pro-
Leu-Asn-Ala-Phe-Lys-Glu-Ser-Lys-Gly-Met-Met-Asn-Asp-Glu (SEQ ID NO.: 21)

### A fusion protein

As used herein, "a fusion protein of the present invention", "a recombinant fusion protein" or "a polypeptide" all refer to the fusion protein of the first aspect of the present invention.

In another preferred embodiment, the structure of the fusion protein is shown as B-C-D (II), in which B is a GLP-1 polypeptide, C is an Fc fragment and D is a CD47 signal polypeptide.

In another preferred embodiment, the fusion protein has an amino acid sequence shown in SEQ ID NO.: 13, 14 or 15.

In another preferred embodiment, an amino acid sequence of a recombinant protein L1-self is shown as SEQ ID NO.: 13.

His-Gly-Glu-Gly-Thr-Phe-Thr-Ser-Asp-Val-Ser-Ser-Tyr-Leu-Glu-Glu-Gln-Ala-Ala-Lys-Glu-Phe-Ile-Ala-
Trp-Leu-Val-Lys-Gly-Gly-Gly-Gly-Gly-Gly-Gly-Ser-Gly-Gly-Gly-Gly-Ser-Gly-Gly-Gly-Gly-Ser-Ala-Glu-
Ser-Lys-Tyr-Gly-Pro-Pro-Cys-Pro-Pro-Cys-Pro-Ala-Pro-Glu-Ala-Ala-Gly-Gly-Pro-Ser-Val-Phe-Leu-
Phe-Pro-Pro-Lys-Pro-Lys-Asp-Thr-Leu-Met-Ile-Ser-Arg-Thr-Pro-Glu-Val-Thr-Cys-Val-Val-Val-Asp-
Val-Ser-Gln-Glu-Asp-Pro-Glu-Val-Gln-Phe-Asn-Trp-Tyr-Val-Asp-Gly-Val-Glu-Val-His-Asn-Ala-Lys-
Thr-Lys-Pro-Arg-Glu-Glu-Gln-Phe-Asn-Ser-Thr-Tyr-Arg-Val-Val-Ser-Val-Leu-Thr-Val-Leu-His-Gln-
Asp-Trp-Leu-Asn-Gly-Lys-Glu-Tyr-Lys-Cys-Lys-Val-Ser-Asn-Lys-Gly-Leu-Pro-Ser-Ser-Ile-Glu-Lys-
Thr-Ile-Ser-Lys-Ala-Lys-Gly-Gln-Pro-Arg-Glu-Pro-Gln-Val-Tyr-Thr-Leu-Pro-Pro-Ser-Gln-Glu-Glu-
Met-Thr-Lys-Asn-Gln-Val-Ser-Leu-Thr-Cys-Leu-Val-Lys-Gly-Phe-Tyr-Pro-Ser-Asp-Ile-Ala-Val-Glu-
Trp-Glu-Ser-Asn-Gly-Gln-Pro-Glu-Asn-Asn-Tyr-Lys-Thr-Thr-Pro-Pro-Val-Leu-Asp-Ser-Asp-Gly-Ser-
Phe-Phe-Leu-Tyr-Ser-Arg-Leu-Thr-Val-Asp-Lys-Ser-Arg-Trp-Gln-Glu-Gly-Asn-Val-Phe-Ser-Cys-Ser-
Val-Met-His-Glu-Ala-Leu-His-Asn-His-Tyr-Thr-Gln-Lys-Ser-Leu-Ser-Leu-Ser-Leu-Gly-Gly-Gly-Gly-
Gly-Gly-Asn-Tyr-Thr-Cys-Glu-Val-Thr-Glu-Leu-Thr-Arg-Glu-Gly-Glu-Thr-Ile-Ile-Glu-Leu-Lys (SEQ
ID NO.: 13)

In another preferred embodiment, an amino acid sequence of a recombinant protein self-L1 is shown as SEQ ID NO.: 14.

Gly-Asn-Tyr-Thr-Cys-Glu-Val-Thr-Glu-Leu-Thr-Arg-Glu-Gly-Glu-Thr-Ile-Ile-Glu-Leu-Lys-Gly-Gly-
Gly-Gly-Gly-His-Gly-Glu-Gly-Thr-Phe-Thr-Ser-Asp-Val-Ser-Ser-Tyr-Leu-Glu-Glu-Gln-Ala-Ala-Lys-
Glu-Phe-Ile-Ala-Trp-Leu-Val-Lys-Gly-Gly-Gly-Gly-Gly-Gly-Gly-Ser-Gly-Gly-Gly-Gly-Ser-Gly-Gly-Gly-
Gly-Ser-Ala-Glu-Ser-Lys-Tyr-Gly-Pro-Pro-Cys-Pro-Pro-Cys-Pro-Ala-Pro-Glu-Ala-Ala-Gly-Gly-Pro-
Ser-Val-Phe-Leu-Phe-Pro-Pro-Lys-Pro-Lys-Asp-Thr-Leu-Met-Ile-Ser-Arg-Thr-Pro-Glu-Val-Thr-Cys-
Val-Val-Val-Asp-Val-Ser-Gln-Glu-Asp-Pro-Glu-Val-Gln-Phe-Asn-Trp-Tyr-Val-Asp-Gly-Val-Glu-Val-His-
Asn-Ala-Lys-Thr-Lys-Pro-Arg-Glu-Glu-Gln-Phe-Asn-Ser-Thr-Tyr-Arg-Val-Val-Ser-Val-Leu-Thr-Val-
Leu-His-Gln-Asp-Trp-Leu-Asn-Gly-Lys-Glu-Tyr-Lys-Cys-Lys-Val-Ser-Asn-Lys-Gly-Leu-Pro-Ser-Ser-
Ile-Glu-Lys-Thr-Ile-Ser-Lys-Ala-Lys-Gly-Gln-Pro-Arg-Glu-Pro-Gln-Val-Tyr-Thr-Leu-Pro-Pro-Ser-Gln-
Glu-Glu-Met-Thr-Lys-Asn-Gln-Val-Ser-Leu-Thr-Cys-Leu-Val-Lys-Gly-Phe-Tyr-Pro-Ser-Asp-Ile-Ala-
Val-Glu-Trp-Glu-Ser-Asn-Gly-Gln-Pro-Glu-Asn-Asn-Tyr-Lys-Thr-Thr-Pro-Pro-Val-Leu-Asp-Ser-Asp-
Gly-Ser-Phe-Phe-Leu-Tyr-Ser-Arg-Leu-Thr-Val-Asp-Lys-Ser-Arg-Trp-Gln-Glu-Gly-Asn-Val-Phe-Ser-
Cys-Ser-Val-Met-His-Glu-Ala-Leu-His-Asn-His-Tyr-Thr-Gln-Lys-Ser-Leu-Ser-Leu-Ser-Leu-Gly-(SEQ
ID NO.: 14)

An amino acid sequence of a recombinant protein L1-self-mu is shown as SEQ ID NO.: 15.

His-Gly-Glu-Gly-Thr-Phe-Thr-Ser-Asp-Val-Ser-Ser-Tyr-Leu-Glu-Glu-Gln-Ala-Ala-Lys-Glu-Phe-Ile-Ala-
Trp-Leu-Val-Arg-Gly-Ala-Gly-Gly-Gly-Gly-Gly-Ser-Gly-Gly-Gly-Gly-Ser-Gly-Gly-Gly-Gly-Ser-Ala-Glu-
Ser-Lys-Tyr-Gly-Pro-Pro-Cys-Pro-Pro-Cys-Pro-Ala-Pro-Glu-Ala-Ala-Gly-Gly-Pro-Ser-Val-Phe-Leu-
Phe-Pro-Pro-Lys-Pro-Lys-Asp-Thr-Leu-Met-Ile-Ser-Arg-Thr-Pro-Glu-Val-Thr-Cys-Val-Val-Val-Asp-
Val-Ser-Gln-Glu-Asp-Pro-Glu-Val-Gln-Phe-Asn-Trp-Tyr-Val-Asp-Gly-Val-Glu-Val-His-Asn-Ala-Lys-
Thr-Lys-Pro-Arg-Glu-Glu-Gln-Phe-Asn-Ser-Thr-Tyr-Arg-Val-Val-Ser-Val-Leu-Thr-Val-Leu-His-Gln-
Asp-Trp-Leu-Asn-Gly-Lys-Glu-Tyr-Lys-Cys-Lys-Val-Ser-Asn-Lys-Gly-Leu-Pro-Ser-Ser-Ile-Glu-Lys-
Thr-Ile-Ser-Lys-Ala-Lys-Gly-Gln-Pro-Arg-Glu-Pro-Gln-Val-Tyr-Thr-Leu-Pro-Pro-Ser-Gln-Glu-Glu-
Met-Thr-Lys-Asn-Gln-Val-Ser-Leu-Thr-Cys-Leu-Val-Lys-Gly-Phe-Tyr-Pro-Ser-Asp-Ile-Ala-Val-Glu-
Trp-Glu-Ser-Asn-Gly-Gln-Pro-Glu-Asn-Asn-Tyr-Lys-Thr-Thr-Pro-Pro-Val-Leu-Asp-Ser-Asp-Gly-Ser-
Phe-Phe-Leu-Tyr-Ser-Arg-Leu-Thr-Val-Asp-Lys-Ser-Arg-Trp-Gln-Glu-Gly-Asn-Val-Phe-Ser-Cys-Ser-
Val-Met-His-Glu-Ala-Leu-His-Asn-His-Tyr-Thr-Gln-Lys-Ser-Leu-Ser-Leu-Ser-Leu-Gly-Gly-Gly-Gly-
Gly-Gly-Asn-Tyr-Thr-Cys-Glu-Val-Thr-Glu-Leu-Thr-Arg-Glu-Gly-Glu-Thr-Ile-Ile-Glu-Leu-Lys (SEQ
ID NO.: 15)

In the present invention, an amino acid sequence of a recombinant protein L1-self is shown as SEQ ID NO.: 11.

His-Gly-Glu-Gly-Thr-Phe-Thr-Ser-Asp-Val-Ser-Ser-Tyr-Leu-Glu-Glu-Gln-Ala-Ala-Lys-Glu-Phe-Ile-Ala-
Trp-Leu-Val-Lys-Gly-Gly-Gly-Gly-Gly-Gly-Gly-Ser-Gly-Gly-Gly-Gly-Ser-Gly-Gly-Gly-Gly-Ser-Ala-Glu-
Ser-Lys-Tyr-Gly-Pro-Pro-Cys-Pro-Pro-Cys-Pro-Ala-Pro-Glu-Ala-Ala-Gly-Gly-Pro-Ser-Val-Phe-Leu-
Phe-Pro-Pro-Lys-Pro-Lys-Asp-Thr-Leu-Met-Ile-Ser-Arg-Thr-Pro-Glu-Val-Thr-Cys-Val-Val-Val-Asp-
Val-Ser-Gln-Glu-Asp-Pro-Glu-Val-Gln-Phe-Asn-Trp-Tyr-Val-Asp-Gly-Val-Glu-Val-His-Asn-Ala-Lys-
Thr-Lys-Pro-Arg-Glu-Glu-Gln-Phe-Asn-Ser-Thr-Tyr-Arg-Val-Val-Ser-Val-Leu-Thr-Val-Leu-His-Gln-
Asp-Trp-Leu-Asn-Gly-Lys-Glu-Tyr-Lys-Cys-Lys-Val-Ser-Asn-Lys-Gly-Leu-Pro-Ser-Ser-Ile-Glu-Lys-
Thr-Ile-Ser-Lys-Ala-Lys-Gly-Gln-Pro-Arg-Glu-Pro-Gln-Val-Tyr-Thr-Leu-Pro-Pro-Ser-Gln-Glu-Glu-
Met-Thr-Lys-Asn-Gln-Val-Ser-Leu-Thr-Cys-Leu-Val-Lys-Gly-Phe-Tyr-Pro-Ser-Asp-Ile-Ala-Val-Glu-
Trp-Glu-Ser-Asn-Gly-Gln-Pro-Glu-Asn-Asn-Tyr-Lys-Thr-Thr-Pro-Pro-Val-Leu-Asp-Ser-Asp-Gly-Ser-
Phe-Phe-Leu-Tyr-Ser-Arg-Leu-Thr-Val-Asp-Lys-Ser-Arg-Trp-Gln-Glu-Gly-Asn-Val-Phe-Ser-Cys-Ser-
Val-Met-His-Glu-Ala-Leu-His-Asn-His-Tyr-Thr-Gln-Lys-Ser-Leu-Ser-Leu-Ser-Leu-Gly (SEQ ID NO.:
11)

In the present invention, an amino acid sequence of a recombinant protein L1-self is shown as SEQ ID NO.: 12.

His-Gly-Glu-Gly-Thr-Phe-Thr-Ser-Asp-Val-Ser-Ser-Tyr-Leu-Glu-Glu-Gln-Ala-Ala-Lys-Glu-Phe-Ile-Ala-
Trp-Leu-Val-Arg-Gly-Ala-Gly-Gly-Gly-Gly-Gly-Ser-Gly-Gly-Gly-Gly-Ser-Gly-Gly-Gly-Gly-Ser-Ala-Glu-
Ser-Lys-Tyr-Gly-Pro-Pro-Cys-Pro-Pro-Cys-Pro-Ala-Pro-Glu-Ala-Ala-Gly-Gly-Pro-Ser-Val-Phe-Leu-
Phe-Pro-Pro-Lys-Pro-Lys-Asp-Thr-Leu-Met-Ile-Ser-Arg-Thr-Pro-Glu-Val-Thr-Cys-Val-Val-Val-Asp-
Val-Ser-Gln-Glu-Asp-Pro-Glu-Val-Gln-Phe-Asn-Trp-Tyr-Val-Asp-Gly-Val-Glu-Val-His-Asn-Ala-Lys-
Thr-Lys-Pro-Arg-Glu-Glu-Gln-Phe-Asn-Ser-Thr-Tyr-Arg-Val-Val-Ser-Val-Leu-Thr-Val-Leu-His-Gln-
Asp-Trp-Leu-Asn-Gly-Lys-Glu-Tyr-Lys-Cys-Lys-Val-Ser-Asn-Lys-Gly-Leu-Pro-Ser-Ser-Ile-Glu-Lys-
Thr-Ile-Ser-Lys-Ala-Lys-Gly-Gln-Pro-Arg-Glu-Pro-Gln-Val-Tyr-Thr-Leu-Pro-Pro-Ser-Gln-Glu-Glu-
Met-Thr-Lys-Asn-Gln-Val-Ser-Leu-Thr-Cys-Leu-Val-Lys-Gly-Phe-Tyr-Pro-Ser-Asp-Ile-Ala-Val-Glu-
Trp-Glu-Ser-Asn-Gly-Gln-Pro-Glu-Asn-Asn-Tyr-Lys-Thr-Thr-Pro-Pro-Val-Leu-Asp-Ser-Asp-Gly-Ser-
Phe-Phe-Leu-Tyr-Ser-Arg-Leu-Thr-Val-Asp-Lys-Ser-Arg-Trp-Gln-Glu-Gly-Asn-Val-Phe-Ser-Cys-Ser-
Val-Met-His-Glu-Ala-Leu-His-Asn-His-Tyr-Thr-Gln-Lys-Ser-Leu-Ser-Leu-Ser-Leu-Gly (SEQ ID NO.:
12)

As used herein, the term "fusion protein" further includes variant forms of the fusion protein with the aforementioned activity (a sequence as shown in SEQ ID NO.: 13, 14 or 15). These variant forms include, but are not limited to, deletions, insertions, and/or substitutions of 1-3 (typically 1-2, more preferably 1) amino acids, and the addition or deletion of one or more (typically within 3, preferably within 2, more preferably within 1) amino acid(s) at a C-terminal and/or an N-terminal. An example is that, substitution with amino acids of similar or analogical properties generally does not alter the function of the protein. Another example is that, the addition or deletion of one or more amino acids at the C-terminal and/or the N-terminal generally does not alter the structure and function of the protein, either. In addition, the term "fusion protein" also includes polypeptides of the present invention in the forms of monomers and multimers as well as linear and non-linear polypeptides (such as cyclic peptides).

The present invention also includes active fragments, derivatives and analogues of the fusion protein. As used herein, the terms "fragments", "derivatives" and "analogues" refer to polypeptides that substantially maintain the function or activity of the fusion protein of this invention. The polypeptide fragment, derivatives or analogues of this invention can be (i) a polypeptide of which one or more conserved or non-conservative amino acid residues (preferably conservative amino acid residues) are substituted, or (ii) a polypeptide with a substituent group in one or more amino acid residues, or (iii) a polypeptide formed by fusing an antigenic peptide with another compound (a compound extending the half-life of the polypeptide, such as polyethylene glycol), or (iv) a polypeptide formed by fusing an additional amino acid sequence to the polypeptide sequence (a fusion protein formed by fusion with a leader sequence, a secretory sequence, or a tag sequence such as 6 x His). According to instructions herein, seasoned technicians are quite familiar with the fragments, derivatives and analogues.

Preferred active derivatives refer to the formation of a polypeptide by replacing at most 3 amino acids, preferably at most 2 amino acids, and more preferably at most 1 amino acid with an amino acid of analogical or similar nature compared to an amino acid sequence in Formula I. These conserved variant polypeptides are preferably generated by amino acid substitutions according to Table A.

**Table A**

| Initial Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogues of the fusion protein of this invention. The difference between the analogues and the polypeptide shown in SEQ ID NO.: 13, 14, or 15 can be a difference in the amino acid sequence, or in modified forms that do not affect the sequence, or both. Analogues also include analogues with residues (such as D-amino acids) different from native L-amino acids, and those with non-native or synthetic amino acids (such as β and γ-amino acids). It should be understood that the polypeptides of this invention are not limited to the representative polypeptides exemplified above.

Modified (usually without altering the primary structure) forms include chemically derived forms of in vivo or in vitro polypeptides such as acetylation or carboxylation. A modification also includes glycosylation, such as polypeptides generated during synthesis and processing of the polypeptides or glycosylation modification in further processing steps. The modification can be accomplished by exposing the polypeptides to an enzyme that performs glycosylation, such as a mammalian glycosylase or deglycosylase. The modified forms also include sequences with phosphorylated amino acid residues (e.g. phosphotyrosine, phosphoserine and phosphothreonine). Polypeptides modified to improve the antiproteolytic properties or optimize solubility properties are also included.

### A nucleic acid coding sequence

The present invention further includes polynucleotides encoding the fusion protein of this invention, and a carrier and a host cell containing the polynucleotides. The present invention also includes a method of treating patient with insulin-independent diabetes, obesity, and other diseases that can benefit from reduction in blood glucose and/or body weight, in which the fusion protein herein is applied.

The present invention also relates to polynucleotides encoding the fusion protein of this invention.

In a preferred embodiment of the present invention, the nucleic acid sequence is shown in SEQ ID NO.: 18, 19 or 20.

In the present invention, a nucleotide sequence encoding a recombinant protein L1-self is shown in SEQ ID NO.: 18.

Cacggcgagggcaccttcacctccgacgtgtcctcctatctcgaggagcaggccgccaaggaattcatcgcctggctggtgaagggcggcggcggtgg
tggtggctccggaggcggcggctctggtggcggtggcagcgctgagtccaaatatggtcccccatgcccaccctgcccagcacctgaggccgccgggg
gaccatcagtcttcctgttccccccaaaacccaaggacactctcatgatctcccggacccctgaggtcacgtgcgtggtggtggacgtgagccaggaag
accccgaggtccagttcaactggtacgtggatggcgtggaggtgcataatgccaagacaaagccgcgggaggagcagttcaacagcacgtaccgtgtgg
tcagcgtcctcaccgtcctgcaccaggactggctgaacggcaaggagtacaagtgcaaggtctccaacaaaggcctcccgtcctccatcgagaaaacc
atctccaaagccaaagggcagccccgagagccacaggtgtacaccctgcccccatcccaggaggagatgaccaagaaccaggtcagcctgacctgcct
ggtcaaaggcttctaccccagcgacatcgccgtggagtgggaaagcaatgggcagccggagaacaactacaagaccacgcctcccgtgctggactccg
acggctccttcttcctctacagcaggctaaccgtggacaagagcaggtggcaggaggggaatgtcttctcatgctccgtgatgcatgaggctctgcacaa
ccactacacacagaagagcctctccctgtctctgggtggtggcggtggcggcaactacacctgcgaggtgaccgagctgacccgcgagggcgagacca
tcatcgagctgaag (SEQ ID NO.: 18)

In the present invention, a nucleotide sequence encoding a recombinant protein self-L1 is shown in SEQ ID NO.: 19.

Ggcaactacacctgcgaggtgaccgagctgacccgcgagggcgagaccatcatcgagctgaagggtggcggtggccacggcgagggcaccttcacct
ccgacgtgtcctcctatctcgaggagcaggccgccaaggaattcatcgcctggctggtgaagggcggcggtggtggctccggaggcggcggct
ctggtggcggtggcagcgctgagtccaaatatggtcccccatgcccaccctgcccagcacctgaggccgccggggaccatcagtcttcctgttcccc
caaaacccaaggacactctcatgatctcccggacccctgaggtcacgtgcgtggtggtggacgtgagccaggaagaccccgaggtccagttcaactggt
acgtggatggcgtggaggtgcataatgccaagacaaagccgcgggaggagcagttcaacagcacgtaccgtgtggtcagcgtcctcaccgtcctgcacc
aggactggctgaacggcaaggagtacaagtgcaaggtctccaacaaaggcctcccgtcctccatcgagaaaaccatctccaaagccaaagggcagccc
cgagagccacaggtgtacaccctgcccccatcccaggaggagatgaccaagaaccaggtcagcctgacctgcctggtcaaaggcttctaccccagcga
catcgccgtggagtgggaaagcaatgggcagccggagaacaactacaagaccacgcctcccgtgctggactccgacggctccttcttcctctacagcag
gctaaccgtggacaagagcaggtggcaggaggggaatgtcttctcatgctccgtgatgcatgaggctctgcacaaccactacacacagaagagcctctc
cctgtctctgggt (SEQ ID NO.: 19)

In the present invention, a nucleotide sequence encoding a recombinant protein L1-self-mu is shown in SEQ ID NO.: 20.

Cacggcgagggcaccttcacctccgacgtgtcctcctatctcgaggagcaggccgccaaggaattcatcgcctggctggtgcgcggcgccggcggtgg
tggtggctccggaggcggcggctctggtggcggtggcagcgctgagtccaaatatggtcccccatgcccaccctgcccagcacctgaggccgccgggg
gaccatcagtcttcctgttccccccaaaacccaaggacactctcatgatctcccggacccctgaggtcacgtgcgtggtggtggacgtgagccaggaag
accccgaggtccagttcaactggtacgtggatggcgtggaggtgcataatgccaagacaaagccgcgggaggagcagttcaacagcacgtaccgtgtgg
tcagcgtcctcaccgtcctgcaccaggactggctgaacggcaaggagtacaagtgcaaggtctccaacaaaggcctcccgtcctccatcgagaaaacc

atctccaaagccaaagggcagccccgagagccacaggtgtacaccctgcccccatcccaggaggagatgaccaagaaccaggtcagcctgacctgcct
ggtcaaaggcttctaccccagcgacatcgccgtggagtgggaaagcaatgggcagccggagaacaactacaagaccacgcctcccgtgctggactccg
acggctccttcttcctctacagcaggctaaccgtggacaagagcaggtggcaggaggggaatgtcttctcatgctccgtgatgcatgaggctctgcacaa
ccactacacacagaagagcctctccctgtctctgggtggtggcggtggcggcaactacacctgcgaggtgaccgagctgacccgcgagggcgagacca
tcatcgagctgaag (SEQ ID NO.: 20)

In the present invention, a nucleotide sequence encoding a recombinant protein L1 is shown in SEQ ID NO.: 16.

Cacggcgagggcaccttcacctccgacgtgtcctcctatctcgaggagcaggccgccaaggaattcatcgcctggctggtgaagggcggcggcggtgg
tggtggctccggaggcggcggctctggtggcggtggcagcgctgagtccaaatatggtcccccatgcccaccctgcccagcacctgaggccgccgggg
gaccatcagtcttcctgttccccccaaaacccaaggacactctcatgatctcccggacccctgaggtcacgtgcgtggtggtggacgtgagccaggaag
accccgaggtccagttcaactggtacgtggatggcgtggaggtgcataatgccaagacaaagccgcgggaggagcagttcaacagcacgtaccgtgtgg
tcagcgtcctcaccgtcctgcaccaggactggctgaacggcaaggagtacaagtgcaaggtctccaacaaaggcctcccgtcctccatcgagaaaacc
atctccaaagccaaagggcagccccgagagccacaggtgtacaccctgcccccatcccaggaggagatgaccaagaaccaggtcagcctgacctgcct
ggtcaaaggcttctaccccagcgacatcgccgtggagtgggaaagcaatgggcagccggagaacaactacaagaccacgcctcccgtgctggactccg
acggctccttcttcctctacagcaggctaaccgtggacaagagcaggtggcaggaggggaatgtcttctcatgctccgtgatgcatgaggctctgcacaa
ccactacacacagaagagcctctccctgtctctgggt (SEQ ID NO.: 16)

A nucleotide sequence encoding a recombinant protein L1-mu is shown in SEQ ID NO.: 17.

Cacggcgagggcaccttcacctccgacgtgtcctcctatctcgaggagcaggccgccaaggaattcatcgcctggctggtgcgcggcgccggcggtgg
tggtggctccggaggcggcggctctggtggcggtggcagcgctgagtccaaatatggtcccccatgcccaccctgcccagcacctgaggccgccgggg
gaccatcagtcttcctgttccccccaaaacccaaggacactctcatgatctcccggacccctgaggtcacgtgcgtggtggtggacgtgagccaggaag
accccgaggtccagttcaactggtacgtggatggcgtggaggtgcataatgccaagacaaagccgcgggaggagcagttcaacagcacgtaccgtgtgg
tcagcgtcctcaccgtcctgcaccaggactggctgaacggcaaggagtacaagtgcaaggtctccaacaaaggcctcccgtcctccatcgagaaaacc
atctccaaagccaaagggcagccccgagagccacaggtgtacaccctgcccccatcccaggaggagatgaccaagaaccaggtcagcctgacctgcct
ggtcaaaggcttctaccccagcgacatcgccgtggagtgggaaagcaatgggcagccggagaacaactacaagaccacgcctcccgtgctggactccg
acggctccttcttcctctacagcaggctaaccgtggacaagagcaggtggcaggaggggaatgtcttctcatgctccgtgatgcatgaggctctgcacaa
ccactacacacagaagagcctctccctgtctctgggt (SEQ ID NO.: 17)

The polynucleotide of this invention can be either in the form of DNA or RNA. The DNA form includes cDNA, genomic DNA, or artificial synthetic DNA. DNA can be single-stranded or doublestranded. DNA can be either a coding strand or a non-coding strand. The coding region sequence encoding a mature polypeptide can be the same as or a degenerate variant of a sequence encoding the polypeptide shown in SEQ ID NO.: 13, 14 or 15. As used herein, the "degenerate variant" in the present invention refers to a nucleic acid sequence that encodes polypeptides as shown in SEQ ID NO.: 13, 14 or 15, but differs in the corresponding coding region sequence.

The nucleotide full-length sequence or the fragments of the polypeptide of this invention can generally be obtained by PCR amplification, recombination or artificial synthetic methods. As for the PCR amplification, primers can be designed according to the published nucleotide sequences, in particular open reading frame sequences, and then amplified to obtain the relevant sequence using commercially available cDNA libraries or cDNA libraries prepared according to conventional methods known to technicians skilled in the art as templates. When the sequence is relatively long, two or more PCR amplifications are usually needed, and then the fragments from each amplification are spliced together in the correct order. At present, DNA sequences encoding the polypeptides of this invention (or its fragments or its derivatives) can be obtained entirely by chemical synthesis. The DNA sequence can then be introduced into various existing DNA molecules (or such as carriers) and cells known in the art.

The present invention also relates to a carrier containing the polynucleotide of this invention, and a host cell genetically engineered using the carrier or the polypeptide coding sequence of this invention. The polynucleotide, the carrier or the host cell can be isolated separately.

As used herein, "isolated" refers to a separation of a substance from its original environment (in the case of a natural substance, the original environment is the natural environment). Polynucleotides and polypeptides of natural status inside living cells are not isolated and purified, but if the same polynucleotides or polypeptides of natural status are separated from other coexisting substances, then they are isolated and purified.

Once obtained, the relevant sequence can be obtained in large quantities by recombination. In the method, this has been done by cloning the relevant sequence into the carrier, then transferring the carrier into a cell, and then isolating the relevant sequence from the proliferated host cells by a conventional method.

In addition, the relevant sequence can be synthesized by an artificial synthetic method, especially when a fragment is short. Typically, a fragment with a long sequence can be obtained by synthesizing several small fragments before connected together.

A method of amplifying DNA/RNA using a PCR technique is preferably used to obtain a gene of the present invention. Primers for PCR can be either appropriately selected according to the sequence information of the present invention disclosed herein or synthesized by a conventional method. DNA/RNA fragments can be amplified via a conventional method through gel electrophoresis isolation and purification.

The present invention also relates to a carrier containing the polynucleotide of this invention, a host cell genetically engineered with the carrier or the protein coding sequence of this invention, and a method of expressing the fusion protein of this invention by a recombinant technique utilizing the host cell.

A host cell expressing the fusion proteins of the present invention can be obtained using the polynucleotide sequence of the present invention by a conventional recombinant DNA technique. Generally the procedure includes introducing the polynucleotide of the third aspect or the carrier of the fourth aspect of this invention into the host cell.

Methods well known to technicians skilled in the art can be used to construct an expression carrier containing a coding DNA sequence and suitable transcription/translation control signals for an enzyme of this invention. The methods include in vitro recombinant DNA technology, DNA synthesis technology, in vivo recombination technology, etc. The DNA sequence can be effectively linked to an appropriate promoter in the expression carrier so as to direct mRNA synthesis. The expression carrier also contains the ribosome binding site and the transcription terminator for translation initiation.

Furthermore, the expression carrier preferably contains one or more selectively labeled genes to provide phenotypic traits for selecting transformed host cells, such as dihydrofolate reductase for eukaryotic cell culture, neomycin resistance and green fluorescent protein (GFP), or tetracycline or ampicillin resistance for Escherichia coli.

A carrier containing the appropriate DNA sequence and an appropriate promoter or a control sequence can be used to transform an appropriate host cell to enable the host cell to express a protein.

A host cell can be a prokaryotic cell, such as a bacterial cell, or a lower eukaryotic cell, such as a yeast cell, or a higher eukaryotic cell, such as a mammalian cell. The representative host cells are: bacterial cells of escherichia coli, bacillus subtilis and genus streptomyces, fungal cells such as pichia pastoris and saccharomyces cerevisiae cells, plant cells, insect cells of drosophila S2 or Sf9; animal cells of CHO, NSO, COS7, or 293 cells, etc.

A transformation of a host cell with recombinant DNA can be performed using conventional techniques well known to technicians skilled in the art. When the host is a prokaryote such as escherichia coli, competent cells capable of absorbing DNA can be harvested after an exponential growth phase after treated with the CaCl₂ method using the steps well known in the art. Another method is use of MgCl₂. The transformation can also be performed by electroporation if needed. When the host is eukaryotic, the following DNA transfection methods can be selected: calcium phosphate coprecipitation, and conventional mechanical methods such as microinjection, electroporation and liposome packaging, etc.

Obtained transformants can be cultured by conventional methods to express the protein encoded by the gene of this invention. Media used in the culture can be selected from various conventional media according to the host cells used. Culturing is performed under conditions suitable for growth of the host cell. When the host cell grows to achieve the appropriate cell density, the selected promoter is induced by a suitable method (such as temperature switching or chemical induction) and the cell is continued to be cultured for a period of time.

The protein used in the above method can be expressed intracellularly or on the cell membrane, or secreted extracellularly. If desired, the protein can be isolated and purified via various methods based on its physical, chemical, and other properties. The methods are well known to seasoned technicians in this field. Examples of the methods include, but are not limited to, conventional renaturation treatment, treatment with protein precipitants (salting-out method), centrifugation, osmosis, overtreatment, ultrasonic treatment, ultracentrifugation, molecular sieve chromatography (gel filtration), adsorption chromatography, ion exchange chromatography, high performance liquid chromatography (HPLC), and various other liquid chromatography techniques and their combinations.

### A pharmaceutical combination

The present invention also provides a pharmaceutical composition. In a preferred embodiment, the composition is a pharmaceutical one containing the fusion protein, and pharmaceutically acceptable carriers, diluents, stabilizers and/or thickeners. The pharmaceutical composition can be prepared as such dosage forms as lyophilized powder, tablets, capsules, syrup, solution or suspension.

"Pharmaceutically acceptable carriers or excipients" refer to one or more compatible solid (s) or liquid filler (s) or gel material (s) which is/are suitable for human use and have sufficient purity and extremely low toxicity. "Compatibility" here refers to mutual blending between components of the composition and active ingredients of this invention without significantly reducing the efficacy of the active ingredients.

The composition can be a liquid or solid, such as a powder, gel or paste. Preferably, the composition is a liquid, and more preferably an injection solution. Approaches of selecting suitable excipients are well known to seasoned technicians in this field.

Examples of the pharmaceutically acceptable carriers are cellulose and its derivatives (such as sodium carboxymethylcellulose, ethylcellulose sodium, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyhydric alcohols (such as propylene glycol, glycerol, mannitol, sorbitol, etc.), emulsifiers (such as Tween®), wetters (such as sodium lauryl sulfate), colorants, flavoring agents, stabilizers, antioxidants, preservatives, pyrogen-free water, etc.

The composition contains physiologically acceptable sterile aqueous or anhydrous solution, dispersion liquid, suspension, or emulsion, and sterile powder for re-dissolving into sterile injection solution or dispersion liquid. Suitable aqueous and nonaqueous carriers, diluents, solvents or excipients include water, ethanol, polyhydric alcohols and its suitable mixtures.

Typically, the substances can be prepared in nontoxic, inert and pharmaceutically acceptable aqueous carrier media, wherein the pH is typically about 5-8, preferably about 6-8 although the pH can vary depending on the properties of the prepared substance and the condition to be treated. The prepared pharmaceutical composition can be administered by a conventional route, including (but not limited to) intraperitoneal, intravenous or topical administration. The pharmaceutical composition is a drug for preventing and/or treating diabetes and related diseases including type 2 diabetes, type 1 diabetes, obesity, serious cardiovascular events and other serious complications in patients with type 2 diabetes.

**Compared with the existing techniques, the present invention mainly has the following advantages:**
(a) The present invention mutates the GLP-1 polypeptide to effectively enhance the biological activity of the GLP-1.
(b) The present invention mutates the Fc fragment to significantly reduce the ADCC effects, decrease potential cytotoxicity and immunogenicity, effectively decrease formation of GLP1-Fc monomers and increase homogeneity and stability of the recombinant fusion proteins.
(c) The fusion protein of the present invention boasts very potent activity with very low EC₅₀ value, thus it can more effectively decrease blood glucose, and significantly prolong the duration of hypoglycemia. With significant duration of efficacy, this invention is significantly superior to the existing products.
(d) The fusion protein of the present invention contains the GLP-1, the Fc fragment and the CD47 signal polypeptide. The fusion protein has been found for the first time to be able to exist as a tetramer, more stable than the homodimeric Fc fusion protein, with effectively prolonged the half-life in vivo. The fusion protein of this invention is significantly superior to the existing products in terms of stability, and is less easily eliminated since the fusion protein has a large molecular weight.
(e) The Fc fragment and the CD47 signal polypeptide in the fusion protein of the present invention are of high human origin, thus inducing less stronger immune response in humans.
(f) The CD47 signal polypeptide (self) in the fusion protein of this invention can mimic the ability of a human CD47, and has a longer half-life in humans, thus achieving a lower dosing frequency.
(g) The coding sequence of the fusion protein of this invention is an optimized sequence and is expressed at a higher level in mammalian cells.

The present invention is further elaborated below through specific embodiments. It should be understood that the embodiments are only intended to illustrate this invention, not to limit the scope of this invention. Experimental methods that do not indicate specific conditions in the following embodiments are generally performed under conventional conditions, such as described in Molecular Cloning: Laboratory Manual by Sambrook et al (New York: Cold Spring Harbor Laboratory Press, 1989) or as recommended by the manufacturer. Unless otherwise stated, the percentages and parts are calculated by weight.

### Embodiment 1: Preparation of GLP-1-Fc recombinant protein

A mammalian cell expression carrier (Bacmam) is selected as the carrier, and tagged proteins (Tag) are selected from 8 histidines (8His) which are tandemly linked to two maltose-binding proteins (MBP) and sumo proteins. The optimized sequence of a DNA of a recombinant protein encoded by artificial synthesis is shown in SEQ ID NOs.: 16-20, and gene segments are cloned into Bacmam-8His-2MBP-sumo using Transfer-PCR (TPCR) (Erijman, Dantes et al. 2011).

Plasmids are transformed into DH10Bac competent cells and then Bacmid DNAs are extracted after screening by blue and white spots. Next, insect cells are transfected to obtain P0 viruses followed by further proliferation of the viruses up to the required volume in the insect cells. The proliferated viruses are expressed in transfected mammalian cells for 4 days.

After 4 days of virus transfection into mammalian cells, the supernatant is collected and centrifuged at 2000 rpm for 20 mins and is loaded slowly onto an Amylose preloaded cylinder. First wash the solution with MBPA buffer solution (Tris 20 mM pH 8.0, NaCl 200 mM, 10% glycerol) until no impurity protein flows out, and then elute the target protein with MBPB buffer solution (Tris 20 mM pH 8.0, NaCl 200 mM, maltose 10 mM, glycerol 10%). Dissecting the target protein from the tagged proteins (Tag) with ULP-1 enzymes, and then separating the target protein from Tag using nickel affinity chromatography.

### Embodiment 2: Modification of GLP-1-Fc recombinant fusion protein GLP-1 and cellular viability evaluation

Native GLP-1 includes two main active forms: GLP-1 (7-36) NH2 and GLP-1 (7-37) (SEQ ID NO.: 1); DPPIV enzymes act between the histidine (His) at position 7 and the alanine (Ala) at position 8. A commonly adopted strategy for extending the half-life of GLP-1 drugs is to mutate the alanine (Ala) at position 8 into the glycine (Gly) (SEQ ID NO.: 2) so as to exclude the possibility of degradation by the DPPIV enzymes.

Studies have shown that the activity of GLP-1 could be increased after the glycine (Gly) at position 22 was mutated into the glutamic acid (Glu) and the arginine (Arg) at position 36 was mutated into the glycine (Gly); meanwhile the GLP-1 fusion protein is also associated with a reduced risk of inducing an immune response (US 2007/0036806 A1). The GLP-1 portion of the marketed drug dulaglutide introduces mutations at positions 8, 22 and 36, respectively (SEQ ID NO.: 3).

The embodiment further mutates the GLP-1 from the lysine (Lys) at position 34 into the arginine (Arg) and the arginine (Arg) at position 36 into the alanine (Ala) or the tyrosine (Val) or the leucine (Leu) (SEQ ID NOs.: 4-7).

The GLP-1 polypeptides with different site mutations (SEQ ID NOs.: 1, 3-7) are synthesized. Culture AD293 cells stably expressing a human GLP-1 receptor (hGLP-1R) and cAMP responsive element (CRE) fusion luciferase (NanoLuc) in high glucose medium (DMEM) containing 10% serum (FBS), then digest it with trypsin at 80% to 90% fusion before inoculating cells into 96-well white clear-bottom plates at 100 µl/well; replace the medium with serum-free medium DMEM after equilibration for 24 h, then measure the luciferase activity after culturing for 4 h by adding polypeptides at different concentrations (prepared in a DMEM medium) . The EC₅₀ value of polypeptide activated hGLP-1R is calculated based on a fitted dose-dependent curve, and the activity of each polypeptide is expressed as a relative value (100% for GLP1 (7-37)). The results show that the GLP-1 polypeptide Gly8-Glu22-Arg34-Ala36-GLP1 (7-37) has the highest activity (Table 1).

**Table 1 Comparison of the Activity of GLP-1 Peptides with Mutations at Different Sites**

| Polypeptides | SEQ ID NO.: | Activity of Activated hGLP-1R |
|---|---|---|
| GLP1(7-37) | 1 | 100% |
| Gly8-Glu22-Gly36-GLP1(7-37) | 3 | 121% |
| Gly8-Glu22-Arg34-Gly36-GLP1(7-37) | 4 | 163% |
| Gly8-Glu22-Arg34-Val36-GLP1(7-37) | 5 | 142% |
| Gly8-Glu22-Arg34-Leu36-GLP1(7-37) | 6 | 113% |
| Gly8-Glu22-Arg34-Ala36-GLP1(7-37) | 7 | 168% |

### Embodiment 3: Introduction of CD47 signals into GLP-1-Fc recombinant fusion protein

The Fc portion of the recombinant protein GLP-1-Fc in the present invention is derived from human IgG4, including a hinge region, CH2 and CH3 domains.Although the ADCC effect caused by IgG4 is weak, potential cytotoxicity still exists.

The present invention introduces two mutations F234A and L235A into the Fc portion of the native human IgG4, which can further effectively reduce the ADCC effects. In addition, this invention also introduces an amino acid mutation S228P, which can effectively lessen the formation of GLP1-Fc monomers while increases the homogeneity and stability of the recombinant fusion protein. Rodriguez et al. design the smallest polypeptide (self) in silico to mimic the ability of a human CD47 to reduce macrophage clearance (self sequence as shown in SEQ ID NO.: 10). The self polypeptide is introduced into the N-terminal and the C-terminal of the L1 protein (dulaglutide, SEQ ID NO.: 11) respectively to obtain the fusion protein self-L1 (SEQ ID NO.: 14) and the fusion protein L1-self (SEQ ID NO.: 13).

The recombinant protein L1-mu (SEQ ID NO.: 12) is obtained by fusing the GLP-1 polypeptide Gly8-Glu22-Arg34-Ala36-GLP1 (7-37) obtained by mutation to the Fc portion (SEQ ID NO.: 9) by the linker peptide P1 (SEQ ID NO.: 8). The self polypeptide is introduced into the C-terminal of the recombinant protein L1-mu to obtain the recombinant protein L1-self-mu (SEQ ID NO.: 15).

The present invention introduces the CD47 signals into the recombinant fusion protein GLP-1-Fc, and the results show that the introduced CD47 signals reduces macrophage clearance of the GLP-1-Fc and thus prolongs its half-life in vivo.

### Embodiment 4: Preparation and Identification of recombinant proteins of L1-self family

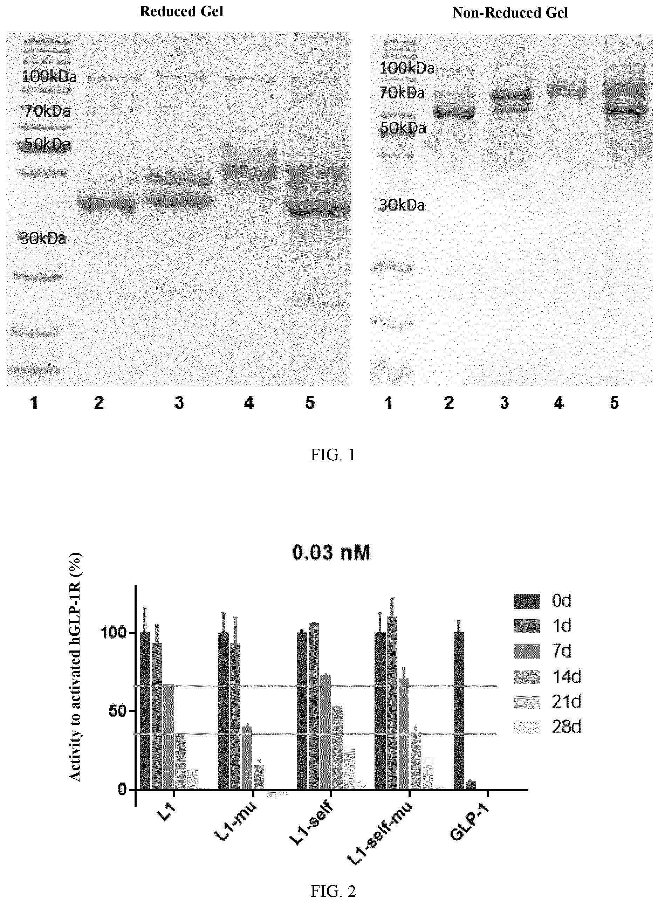

In conjunction with the results of embodiment 2 and embodiment 3, a family of recombinant proteins are prepared: positive control L1 (SEQ ID NO.: 11), LI-mu (SEQ ID NO.: 12), L1-self (SEQ ID NO.: 13), and L1-self-mu (SEQ ID NO.: 15). Proteins with high expression level and purity are prepared according to the method of embodiment 1 (as shown in Fig.1). The results of non-reduced gel figure show that all four recombinant proteins form disulfide bonds.

The aggregated morphology of the proteins is identified by HPLC or molecular sieve methods and the positive control protein L1 is confirmed to be a dimer. The Protein L1 and a protein reference standard with known molecular weight are separated and tested by HPLC, respectively, with the column Zenix-C SEC-300.

Results are shown in Table 2, and the peak time of L1 is 23.692 min. Compared with the protein reference standard, the molecular weight of L1 is between 75 and 158 kD, while the theoretical molecular weight of the L1 monomer is 29.8 kD; in consideration about some modifications in the expression of L1 in mammalian cells, the actual molecular weight is relatively large, so it can be concluded that L1 is present as a dimer.

**Table 2 Peak Time of Protein in Zenix-C SEC-300 Column**

| Protein | Peak Time (min) | Molecular Weight (kD) |
|---|---|---|
| L1 | 23.692 | 29.8 |
| Protein Reference Standard 1 | 25.377 | 43 |
| Protein Reference Standard 2 | 25.011 | 75 |
| Protein Reference Standard 3 | 22.526 | 158 |
| Protein Reference Standard 4 | 19.651 | 440 |

The protein L1-self-mu and the protein reference standard with known molecular weight are separated and detected by HPLC, respectively, with the column TSKgel G4000SW. Results are shown in Table 3, and the peak time of L1-self-mu is 20.357 min. Compared with the protein reference standard, the molecular weight of L1-self-mu is between 158 and 440 kD, while the theoretical molecular weight of L1-self-mu monomer is only 32.5 kD, so it can be concluded that L1-self-mu is present as a tetramer.

**Table 3 Peak Time of Protein in TSKgel G4000SW Column**

| Protein | Peak Time (min) | Molecular Weight (kD) |
|---|---|---|
| L1-self-mu | 20.357 | 32.5 |
| Protein Reference Standard 1 | 21.402 | 43 |
| Protein Reference Standard 2 | 21.174 | 75 |
| Protein Reference Standard 3 | 20.900 | 158 |
| Protein Reference Standard 4 | 19.320 | 440 |

The L1-mu and the L1-self are identified using a molecular sieve method, and the retention volumes of the L1-mu and the L1-self in the Superdex 200 column are 13.39 mL and 10.21 mL, respectively, and the theoretical molecular weights of their monomers are 29.9 kD and 32.4 kD, respectively. The retention volumes of the protein reference standards are 14.34 ml (66 kD) and 10.94 ml (440 kD), so it can be concluded that the L1-mu and the L1-self exist as dimers and tetramers, respectively.

The aggregated morphology of the protein self-L1 is identified using the same experimental method, and the result indicates that the protein self-L1 also exist as tetramers. The above results indicate that the protein conformation changes after the introduction of the CD47 signals (L1-self and L1-self-mu). Similar to L1, the Fc fragment of the L1-self and the L1-self-mu forms into dimers through disulfide bonds; in contrast to L1, dimers of the L1-self, the self-L1 protein and the L1-self-mu can continue to form tetramers. L1-self and L1-self-mu are found to be more stable in subsequent trial validation. The trial data demonstrates that no disulfide bond is formed among the dimers of the L1-self, the self-L1 protein and the L1-self-mu, indicating that the tetramers are formed by non-covalent bonds such as hydrophobic action.

### Embodiment 5: Evaluation of activity of L1-self family recombinant proteins in activating hGLP-1R

The activities of the L1, the L1-mu, the L1-self, the self-L1 and the L1-self-mu in activating the hGLP-1R are evaluated in AD293 cells that stably express the hGLP-1R and the CRE fusion luciferase (NanoLuc). Using the same approach as that mentioned in embodiment 2, including diluting the protein with DMEM to different concentrations (100 nM to 0.1 pM gradient dilution, the molecular weight of the protein calculated as the molecular weight of the monomer), then test the activity of the luciferase 4 h after protein treatment.

Results are shown in Table 5, after partial mutation of the L1 polypeptide (L1-mu), no significant improvement is found in the cellular activity of the L1-self and the L1 protein, and the activity of self-L1 (self polypeptide is located at the N-terminal of the L1 protein) is decreased. However, the cellular activity of the L1-self-mu is enhanced, higher than that of the L1 protein.

**Table 2 Activity of L1-self Family Recombinant Proteins in Activating hGLP-1R**

| Recombinant Protein | SEQ ID NO,: | EC₅₀ (pM) of Activity to Activate hGLP-1R |
|---|---|---|
| L1 | 11 | 23.89 |
| L1-mu | 12 | 83.58 |
| L1-self | 13 | 45.64 |
| self-L 1 | 14 | 24270 |
| L1-self-mu | 15 | 16.93 |

| | | |
|---|---|---|
| Note: The data are shown in mean values and the tests have been run in triplicates. | | |

### Embodiment 6: Evaluation on L1-self family recombinant proteins in rat serum in vitro

This embodiment evaluates the stability in rat serum in vitro as follows: first dilute the protein to 10 nM with rat serum and then incubate the solution in a 5% CO₂ incubator at 37°C for 0, 1, 7, 14, 21 and 28 days, then test the activity of the protein in activating the hGLP-1R at a concentration of 0.03 nM. The decay rate of protein activity indirectly reflects the stability of the protein in the serum.

Results are shown in Fig.2; the negative control GLP-1 is very unstable in the serum, with a 96% decrease in activity after one day of incubation, while the activities of the four recombinant proteins remains basically unchanged after one day of incubation in the serum. After 7 days of incubation, changes in the activities show significant differences, with a 33.3% decrease in the positive control L1, a 60.4% decrease in the L1-mu, and 27.8% and 29.9% decreases in the L1-self and the L1-self-mu, respectively, indicating that the L1-self and the L1-self-mu are much more stable than the positive control L1 in the rat serum in vitro.

### Embodiment 7: Hypoglycemic activity of L1-self family recombinant proteins in animals

Male C57BL/KsJ mice of 6 weeks old are randomized into a vehicle control group, a positive drug group and a test protein group according to body weights and blood glucose levels, and are subcutaneously (s.c.) injected with corresponding drugs. An intraperitoneal glucose tolerance test (IPGTT) is performed after dosing for a period of time following the steps as follows: fast the mice for 6 h, collect blood samples from tail veins of the mice in each group before the test, measure the blood glucose levels with a Roche ACCU-CHEK blood glucose meter and record the results at a -60 min time point; measure blood glucose levels again at 0 min in each group before intraperitoneally injecting 2 g/kg glucose (body weight), afterwards measure and record blood glucose levels of mice in each group at 15 min, 30 min, 60 min and 120 min. An IPGTT curve is plotted based on the blood glucose levels to calculate the areas under curve (AUC0-120min (mM*min)), and the differences are statistically analyzed using the t-test.

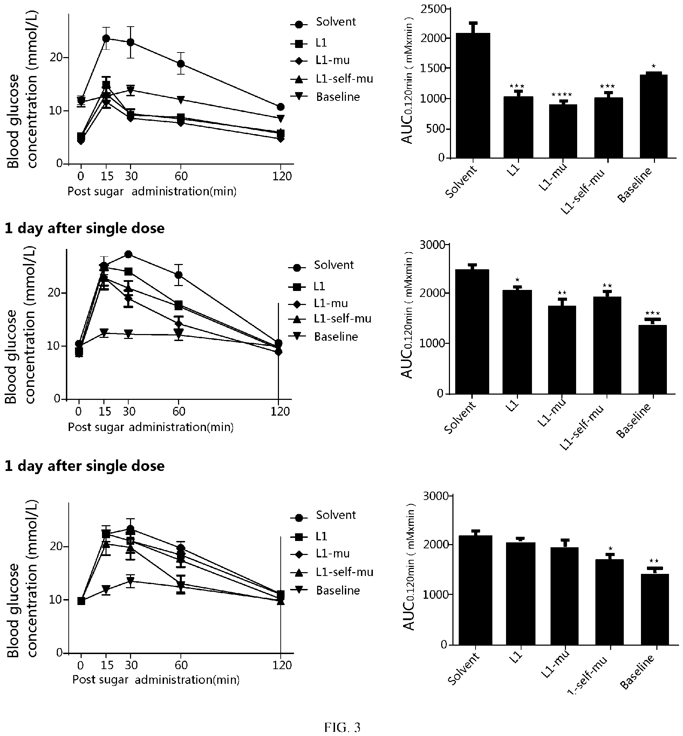

Results are shown in Fig.3, and the L1-self-mu has a longer hypoglycemic duration than the L1. Seven days after a single dose, the area under curve (AUC) in the blood glucose decreases by 14.8% in the positive drug L1 group and decreases by 28.2% and 20% in the L1-mu and L1-self-mu groups, respectively, as compared with the solvent control group. Ten days post-dose, no significant decrease is found in AUC in the L1 and L1-mu groups, and significant hypoglycemic activity is still observed in the L1-self-mu group, indicating that the hypoglycemic duration of the L1-self-mu in animals last longer than that of the positive drug L1. Despite low protein expression level and less animals in L1-self group, its hypoglycemic activity and duration are similar to that of the L1-self-mu.

### Embodiment 8

Except replacement of the self polypeptide (SEQ ID NO.: 10) with proteins or polypeptides with CD47 functions (human CD47 proteins as shown in SEQ ID NO.: 21), other methods employed are the same as that described in embodiments 1-7. The results show that the stability of the fusion protein resulting from the introduction of human CD47 proteins is significantly increased and the half-life is effectively prolonged.

All the literatures mentioned in the present inventions are cited as references in this application, as the way that each literature is individually cited as a reference. Furthermore, it should be understood that after reading the instructions of the present invention, seasoned technicians in this field can make various changes or modifications to the present invention accordingly, and the equivalent forms are also within the scope defined by the claims attached to the application.

## Claims

1. A fusion protein with a structure as shown in Formula I below:
A-B-C-D (I)
wherein each "-" represents independently a linker peptide or a peptide bond;
A is absent or a CD47 signal polypeptide;
B is a GLP-1 polypeptide;
C is an Fc fragment;
D is absent or a CD47 signal polypeptide; and
A and D are not absent at the same time.

2. This fusion protein of claim 1, **characterized by** the absence of the A and presence of a CD47 signal polypeptide of the D.

3. This fusion protein of claim 1, **characterized by** the CD47 signal polypeptide that contains a core sequence as shown in SEQ ID NO.: 10.

4. This fusion protein of claim 1, **characterized by** an amino acid sequence as shown in SEQ ID NO.: 13, 14 or 15.

5. An oligomer, **characterized by** the presence of this fusion protein of claim 1.

6. An isolated polynucleotide, which encodes this fusion protein of claim 1.

7. A carrier, **characterized by** the presence of the polynucleotide of claim 6.

8. A host cell, **characterized by** the presence of the carrier of claim 7, or of the exogenous polynucleotide of claim 6 in chromosomes, or the expression of the fusion protein of claim 1 or the oligomer of claim 5.

9. A pharmaceutical composition, **characterized by** the presence of this fusion protein of claim 1 or the oligomer of claim 5, and of pharmaceutically acceptable carriers or excipients.

10. The use of this fusion protein of claim 1, the oligomer of claim 5, the polynucleotide of claim 6, the carrier of claim 7, and the host cell of claim 8 is for preparing a drug or formulation which prevents and/or treats diabetes, obesity, and other diseases that can benefit from reduction in blood glucose and/or body weight.


Sequence Listing

<110> Zhejiang Palo Alto Pharmaceuticals, Inc.

<120> Long-Acting Recombinant GLP1-Fc-CD47 Protein and Preparation and Use
Thereof

<130> P2018-1023

<150> CN201811039238.X
<151> 2018-09-06

<160> 21

<170> PatentIn version 3.5

<210> 1
<211> 31
<212> PRT
<213> Homo sapiens

<400> 1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 2
<211> 31
<212> PRT
<213> Artificial sequence

<220>
<223> GLP-1 mutant

<400> 2

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 3
<211> 31
<212> PRT
<213> Artificial sequence

<220>
<223> GLP-1 mutant

<400> 3

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Gly Gly
            20                  25                  30

<210>  4
<211>  31
<212>  PRT
<213>  Artificial sequence

<220>
<223>  GLP-1 mutant

<400>  4

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Gly Gly
            20                  25                  30

<210>  5
<211>  31
<212>  PRT
<213>  Artificial sequence

<220>
<223>  GLP-1 mutant

<400>  5

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Val Gly
            20                  25                  30

<210>  6
<211>  31
<212>  PRT
<213>  Artificial sequence

<220>
<223>  GLP-1 mutant

<400>  6

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Leu Gly
            20                  25                  30

<210>  7
<211>  31
<212>  PRT
<213>  Artificial sequence

<220>
<223>  GLP-1 mutant

<400>  7

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Ala Gly
            20                  25                  30

<210>  8
<211>  15
<212>  PRT
<213>  Artificial sequence

<220>
<223>  Connecting peptide P1

<400>  8

Gly Gly Gly Gly Ser Gly Gly Gly Gly Ser Gly Gly Gly Gly Ser
1               5                   10                  15

<210>  9
<211>  229
<212>  PRT
<213>  Artificial sequence

<220>
<223>  Fc fragment

<400>  9

Ala Glu Ser Lys Tyr Gly Pro Pro Cys Pro Pro Cys Pro Ala Pro Glu
1               5                   10                  15

Ala Ala Gly Gly Pro Ser Val Phe Leu Phe Pro Pro Lys Pro Lys Asp
            20                  25                  30

Thr Leu Met Ile Ser Arg Thr Pro Glu Val Thr Cys Val Val Val Asp
        35                  40                  45

Val Ser Gln Glu Asp Pro Glu Val Gln Phe Asn Trp Tyr Val Asp Gly
    50                  55                  60

Val Glu Val His Asn Ala Lys Thr Lys Pro Arg Glu Glu Gln Phe Asn
65                  70                  75                  80

Ser Thr Tyr Arg Val Val Ser Val Leu Thr Val Leu His Gln Asp Trp
                85                  90                  95

Leu Asn Gly Lys Glu Tyr Lys Cys Lys Val Ser Asn Lys Gly Leu Pro
            100                 105                 110

Ser Ser Ile Glu Lys Thr Ile Ser Lys Ala Lys Gly Gln Pro Arg Glu
        115                 120                 125

Pro Gln Val Tyr Thr Leu Pro Pro Ser Gln Glu Glu Met Thr Lys Asn
    130                 135                 140

Gln Val Ser Leu Thr Cys Leu Val Lys Gly Phe Tyr Pro Ser Asp Ile
145                 150                 155                 160

Ala Val Glu Trp Glu Ser Asn Gly Gln Pro Glu Asn Asn Tyr Lys Thr
                165                 170                 175

Thr Pro Pro Val Leu Asp Ser Asp Gly Ser Phe Phe Leu Tyr Ser Arg
            180                 185                 190

Leu Thr Val Asp Lys Ser Arg Trp Gln Glu Gly Asn Val Phe Ser Cys
        195                 200                 205

Ser Val Met His Glu Ala Leu His Asn His Tyr Thr Gln Lys Ser Leu
    210                 215                 220

Ser Leu Ser Leu Gly
225

<210>  10
<211>  21
<212>  PRT
<213>  Artificial sequence

<220>
<223>  CD47 signal polypeptide core sequence

<400>  10

Gly Asn Tyr Thr Cys Glu Val Thr Glu Leu Thr Arg Glu Gly Glu Thr
1               5                   10                  15

Ile Ile Glu Leu Lys
            20

<210>  11
<211>  275
<212>  PRT
<213>  Artificial sequence

<220>
<223>  Recombinant protein L1

<400>  11

His Gly Glu Gly Thr Phe Thr Ser Asp Val Ser Ser Tyr Leu Glu Glu

1               5                   10                  15

Gln Ala Ala Lys Glu Phe Ile Ala Trp Leu Val Lys Gly Gly Gly Gly
            20                  25                  30

Gly Gly Gly Ser Gly Gly Gly Gly Ser Gly Gly Gly Gly Ser Ala Glu
        35                  40                  45

Ser Lys Tyr Gly Pro Pro Cys Pro Pro Cys Pro Ala Pro Glu Ala Ala
    50                  55                  60

Gly Gly Pro Ser Val Phe Leu Phe Pro Pro Lys Pro Lys Asp Thr Leu
65                  70                  75                  80

Met Ile Ser Arg Thr Pro Glu Val Thr Cys Val Val Val Asp Val Ser
                85                  90                  95

Gln Glu Asp Pro Glu Val Gln Phe Asn Trp Tyr Val Asp Gly Val Glu
            100                 105                 110

Val His Asn Ala Lys Thr Lys Pro Arg Glu Glu Gln Phe Asn Ser Thr
        115                 120                 125

Tyr Arg Val Val Ser Val Leu Thr Val Leu His Gln Asp Trp Leu Asn
    130                 135                 140

Gly Lys Glu Tyr Lys Cys Lys Val Ser Asn Lys Gly Leu Pro Ser Ser
145                 150                 155                 160

Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val
            180                 185                 190

Ser Leu Thr Cys Leu Val Lys Gly Phe Tyr Pro Ser Asp Ile Ala Val
        195                 200                 205

Glu Trp Glu Ser Asn Gly Gln Pro Glu Asn Asn Tyr Lys Thr Thr Pro
    210                 215                 220

Pro Val Leu Asp Ser Asp Gly Ser Phe Phe Leu Tyr Ser Arg Leu Thr
225                 230                 235                 240

Val Asp Lys Ser Arg Trp Gln Glu Gly Asn Val Phe Ser Cys Ser Val
                245                 250                 255

Met His Glu Ala Leu His Asn His Tyr Thr Gln Lys Ser Leu Ser Leu
            260                 265                 270

Ser Leu Gly
        275

<210>  12
<211>  275
<212>  PRT
<213>  Artificial sequence

<220>
<223>  Recombinant protein L1-mu

<400>  12

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Ala Gly Gly
            20                  25                  30

Gly Gly Gly Ser Gly Gly Gly Gly Ser Gly Gly Gly Gly Ser Ala Glu
        35                  40                  45

Ser Lys Tyr Gly Pro Pro Cys Pro Pro Cys Pro Ala Pro Glu Ala Ala
    50                  55                  60

Gly Gly Pro Ser Val Phe Leu Phe Pro Pro Lys Pro Lys Asp Thr Leu
65                  70                  75                  80

Met Ile Ser Arg Thr Pro Glu Val Thr Cys Val Val Val Asp Val Ser
                85                  90                  95

Gln Glu Asp Pro Glu Val Gln Phe Asn Trp Tyr Val Asp Gly Val Glu
            100                 105                 110

Val His Asn Ala Lys Thr Lys Pro Arg Glu Glu Gln Phe Asn Ser Thr
        115                 120                 125

Tyr Arg Val Val Ser Val Leu Thr Val Leu His Gln Asp Trp Leu Asn
    130                 135                 140

Gly Lys Glu Tyr Lys Cys Lys Val Ser Asn Lys Gly Leu Pro Ser Ser
145                 150                 155                 160

Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val

180                 185                 190

Ser Leu Thr Cys Leu Val Lys Gly Phe Tyr Pro Ser Asp Ile Ala Val
        195                 200                 205

Glu Trp Glu Ser Asn Gly Gln Pro Glu Asn Asn Tyr Lys Thr Thr Pro
    210                 215                 220

Pro Val Leu Asp Ser Asp Gly Ser Phe Phe Leu Tyr Ser Arg Leu Thr
225                 230                 235                 240

Val Asp Lys Ser Arg Trp Gln Glu Gly Asn Val Phe Ser Cys Ser Val
                245                 250                 255

Met His Glu Ala Leu His Asn His Tyr Thr Gln Lys Ser Leu Ser Leu
            260                 265                 270

Ser Leu Gly
        275

<210>  13
<211>  300
<212>  PRT
<213>  Artificial sequence

<220>
<223> Recombinant protein L1-self

<400>  13

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Gly Gly Gly
            20                  25                  30

Gly Gly Gly Ser Gly Gly Gly Gly Ser Gly Gly Gly Gly Ser Ala Glu
        35                  40                  45

Ser Lys Tyr Gly Pro Pro Cys Pro Pro Cys Pro Ala Pro Glu Ala Ala
    50                  55                  60

Gly Gly Pro Ser Val Phe Leu Phe Pro Pro Lys Pro Lys Asp Thr Leu
65                  70                  75                  80

Met Ile Ser Arg Thr Pro Glu Val Thr Cys Val Val Val Asp Val Ser
                85                  90                  95

Gln Glu Asp Pro Glu Val Gln Phe Asn Trp Tyr Val Asp Gly Val Glu
            100                 105                 110

Val His Asn Ala Lys Thr Lys Pro Arg Glu Glu Gln Phe Asn Ser Thr
        115                 120                 125

Tyr Arg Val Val Ser Val Leu Thr Val Leu His Gln Asp Trp Leu Asn
    130                 135                 140

Gly Lys Glu Tyr Lys Cys Lys Val Ser Asn Lys Gly Leu Pro Ser Ser
145                 150                 155                 160

Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val
            180                 185                 190

Ser Leu Thr Cys Leu Val Lys Gly Phe Tyr Pro Ser Asp Ile Ala Val
        195                 200                 205

Glu Trp Glu Ser Asn Gly Gln Pro Glu Asn Asn Tyr Lys Thr Thr Pro
    210                 215                 220

Pro Val Leu Asp Ser Asp Gly Ser Phe Phe Leu Tyr Ser Arg Leu Thr
225                 230                 235                 240

Val Asp Lys Ser Arg Trp Gln Glu Gly Asn Val Phe Ser Cys Ser Val
                245                 250                 255

Met His Glu Ala Leu His Asn His Tyr Thr Gln Lys Ser Leu Ser Leu
            260                 265                 270

Ser Leu Gly Gly Gly Gly Gly Gly Asn Tyr Thr Cys Glu Val Thr Glu
        275                 280                 285

Leu Thr Arg Glu Gly Glu Thr Ile Ile Glu Leu Lys
    290                 295                 300

<210>  14
<211>  301
<212>  PRT
<213>  Artificial sequence

<220>
<223>  Recombinant protein self-L1

<400>  14
   Gly Asn Tyr Thr Cys Glu Val Thr Glu Leu Thr Arg Glu Gly Glu Thr
   1               5                   10                  15
   Ile Ile Glu Leu Lys Gly Gly Gly Gly Gly His Gly Glu Gly Thr Phe
               20                  25                  30
   Thr Ser Asp Val Ser Ser Tyr Leu Glu Glu Gln Ala Ala Lys Glu Phe
           35                  40                  45
   Ile Ala Trp Leu Val Lys Gly Gly Gly Gly Gly Gly Gly Ser Gly Gly
       50                  55                  60
   Gly Gly Ser Gly Gly Gly Gly Ser Ala Glu Ser Lys Tyr Gly Pro Pro
   65                  70                  75                  80
   Cys Pro Pro Cys Pro Ala Pro Glu Ala Ala Gly Gly Pro Ser Val Phe
                   85                  90                  95
   Leu Phe Pro Pro Lys Pro Lys Asp Thr Leu Met Ile Ser Arg Thr Pro
               100                 105                 110
   Glu Val Thr Cys Val Val Val Asp Val Ser Gln Glu Asp Pro Glu Val
           115                 120                 125
   Gln Phe Asn Trp Tyr Val Asp Gly Val Glu Val His Asn Ala Lys Thr
       130                 135                 140
   Lys Pro Arg Glu Glu Gln Phe Asn Ser Thr Tyr Arg Val Val Ser Val
   145                 150                 155                 160
   Leu Thr Val Leu His Gln Asp Trp Leu Asn Gly Lys Glu Tyr Lys Cys
                   165                 170                 175
   Lys Val Ser Asn Lys Gly Leu Pro Ser Ser Ile Glu Lys Thr Ile Ser
               180                 185                 190
   Lys Ala Lys Gly Gln Pro Arg Glu Pro Gln Val Tyr Thr Leu Pro Pro
           195                 200                 205
   Ser Gln Glu Glu Met Thr Lys Asn Gln Val Ser Leu Thr Cys Leu Val
       210                 215                 220
   Lys Gly Phe Tyr Pro Ser Asp Ile Ala Val Glu Trp Glu Ser Asn Gly
   225                 230                 235                 240
   Gln Pro Glu Asn Asn Tyr Lys Thr Thr Pro Pro Val Leu Asp Ser Asp
                   245                 250                 255
   Gly Ser Phe Phe Leu Tyr Ser Arg Leu Thr Val Asp Lys Ser Arg Trp
               260                 265                 270
   Gln Glu Gly Asn Val Phe Ser Cys Ser Val Met His Glu Ala Leu His
           275                 280                 285
   Asn His Tyr Thr Gln Lys Ser Leu Ser Leu Ser Leu Gly
       290                 295                 300

<210>  15
<211>  300
<212>  PRT
<213>  Artificial sequence

<220>
<223>  Recombinant protein L1-self-mu

<400>  15

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Arg Gly Ala Gly Gly
            20                  25                  30

Gly Gly Gly Ser Gly Gly Gly Gly Ser Gly Gly Gly Gly Ser Ala Glu
        35                  40                  45

Ser Lys Tyr Gly Pro Pro Cys Pro Pro Cys Pro Ala Pro Glu Ala Ala
    50                  55                  60

Gly Gly Pro Ser Val Phe Leu Phe Pro Pro Lys Pro Lys Asp Thr Leu
65                  70                  75                  80

Met Ile Ser Arg Thr Pro Glu Val Thr Cys Val Val Val Asp Val Ser
                85                  90                  95

Gln Glu Asp Pro Glu Val Gln Phe Asn Trp Tyr Val Asp Gly Val Glu
            100                 105                 110

Val His Asn Ala Lys Thr Lys Pro Arg Glu Glu Gln Phe Asn Ser Thr
        115                 120                 125

Tyr Arg Val Val Ser Val Leu Thr Val Leu His Gln Asp Trp Leu Asn
    130                 135                 140

Gly Lys Glu Tyr Lys Cys Lys Val Ser Asn Lys Gly Leu Pro Ser Ser
145                 150                 155                 160

Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val
            180                 185                 190

Ser Leu Thr Cys Leu Val Lys Gly Phe Tyr Pro Ser Asp Ile Ala Val
        195                 200                 205

Glu Trp Glu Ser Asn Gly Gln Pro Glu Asn Asn Tyr Lys Thr Thr Pro
    210                 215                 220

Pro Val Leu Asp Ser Asp Gly Ser Phe Phe Leu Tyr Ser Arg Leu Thr
225                 230                 235                 240

Val Asp Lys Ser Arg Trp Gln Glu Gly Asn Val Phe Ser Cys Ser Val
                245                 250                 255

Met His Glu Ala Leu His Asn His Tyr Thr Gln Lys Ser Leu Ser Leu
            260                 265                 270

Ser Leu Gly Gly Gly Gly Gly Gly Asn Tyr Thr Cys Glu Val Thr Glu
        275                 280                 285

Leu Thr Arg Glu Gly Glu Thr Ile Ile Glu Leu Lys
    290                 295                 300

<210>  16
<211>  825
<212>  DNA
<213>  Artificial sequence

<220>
<223>  Recombinant protein L1

<400>  16
cacggcgagg gcaccttcac ctccgacgtg tcctcctatc tcgaggagca ggccgccaag      60

gaattcatcg cctggctggt gaagggcggc ggcggtggtg gtggctccgg aggcggcggc     120

tctggtggcg gtggcagcgc tgagtccaaa tatggtcccc catgcccacc ctgcccagca     180

cctgaggccg ccgggggacc atcagtcttc ctgttccccc caaaacccaa ggacactctc     240

atgatctccc ggacccctga ggtcacgtgc gtggtggtgg acgtgagcca ggaagacccc     300

gaggtccagt tcaactggta cgtggatggc gtggaggtgc ataatgccaa gacaaagccg     360

cgggaggagc agttcaacag cacgtaccgt gtggtcagcg tcctcaccgt cctgcaccag     420

gactggctga acggcaagga gtacaagtgc aaggtctcca acaaaggcct cccgtcctcc     480

atcgagaaaa ccatctccaa agccaaaggg cagccccgag agccacaggt gtacaccctg     540

cccccatccc aggaggagat gaccaagaac caggtcagcc tgacctgcct ggtcaaaggc     600

ttctacccca gcgacatcgc cgtggagtgg gaaagcaatg ggcagccgga gaacaactac     660

aagaccacgc ctcccgtgct ggactccgac ggctccttct tcctctacag caggctaacc 720

gtggacaaga gcaggtggca ggaggggaat gtcttctcat gctccgtgat gcatgaggct 780

ctgcacaacc actacacaca gaagagcctc tccctgtctc tgggt 825

<210> 17
<211> 825
<212> DNA
<213> Artificial sequence

<220>
<223> Recombinant protein L1-mu

<400> 17
cacggcgagg gcaccttcac ctccgacgtg tcctcctatc tcgaggagca ggccgccaag 60

gaattcatcg cctggctggt gcgcggcgcc ggcggtggtg gtggctccgg aggcggcggc 120

tctggtggcg gtggcagcgc tgagtccaaa tatggtcccc catgcccacc ctgcccagca 180

cctgaggccg ccgggggacc atcagtcttc ctgttccccc caaaacccaa ggacactctc 240

atgatctccc ggacccctga ggtcacgtgc gtggtggtgg acgtgagcca ggaagacccc 300

gaggtccagt tcaactggta cgtggatggc gtggaggtgc ataatgccaa gacaaagccg 360

cgggaggagc agttcaacag cacgtaccgt gtggtcagcg tcctcaccgt cctgcaccag 420

gactggctga acggcaagga gtacaagtgc aaggtctcca acaaaggcct cccgtcctcc 480

atcgagaaaa ccatctccaa agccaaaggg cagccccgag agccacaggt gtacaccctg 540

cccccatccc aggaggagat gaccaagaac caggtcagcc tgacctgcct ggtcaaaggc 600

ttctacccca gcgacatcgc cgtggagtgg gaaagcaatg ggcagccgga gaacaactac 660

aagaccacgc ctcccgtgct ggactccgac ggctccttct tcctctacag caggctaacc 720

gtggacaaga gcaggtggca ggaggggaat gtcttctcat gctccgtgat gcatgaggct 780

ctgcacaacc actacacaca gaagagcctc tccctgtctc tgggt 825

<210> 18
<211> 900
<212> DNA
<213> Artificial sequence

<220>
<223> Recombinant protein L1-self

<400> 18
cacggcgagg gcaccttcac ctccgacgtg tcctcctatc tcgaggagca ggccgccaag 60

gaattcatcg cctggctggt gaagggcggc ggcggtggtg gtggctccgg aggcggcggc 120

tctggtggcg gtggcagcgc tgagtccaaa tatggtcccc catgcccacc ctgcccagca 180

cctgaggccg ccgggggacc atcagtcttc ctgttccccc caaaacccaa ggacactctc 240

atgatctccc ggacccctga ggtcacgtgc gtggtggtgg acgtgagcca ggaagacccc 300

gaggtccagt tcaactggta cgtggatggc gtggaggtgc ataatgccaa gacaaagccg 360

cgggaggagc agttcaacag cacgtaccgt gtggtcagcg tcctcaccgt cctgcaccag 420

gactggctga acggcaagga gtacaagtgc aaggtctcca acaaaggcct cccgtcctcc 480

atcgagaaaa ccatctccaa agccaaaggg cagccccgag agccacaggt gtacaccctg 540

cccccatccc aggaggagat gaccaagaac caggtcagcc tgacctgcct ggtcaaaggc 600

ttctacccca gcgacatcgc cgtggagtgg gaaagcaatg ggcagccgga gaacaactac 660

aagaccacgc ctcccgtgct ggactccgac ggctccttct tcctctacag caggctaacc 720

gtggacaaga gcaggtggca ggaggggaat gtcttctcat gctccgtgat gcatgaggct 780

ctgcacaacc actacacaca gaagagcctc tccctgtctc tgggtggtgg cggtggcggc 840

aactacacct gcgaggtgac cgagctgacc cgcgagggcg agaccatcat cgagctgaag 900

<210> 19
<211> 900
<212> DNA
<213> Artificial sequence

<220>
<223> Recombinant protein self-L1

<400> 19
ggcaactaca cctgcgaggt gaccgagctg acccgcgagg gcgagaccat catcgagctg 60

aagggtggcg gtggccacgg cgagggcacc ttcacctccg acgtgtcctc ctatctcgag 120

gagcaggccg ccaaggaatt catcgcctgg ctggtgaagg gcggcggcgg tggtggtggc 180

tccggaggcg gcggctctgg tggcggtggc agcgctgagt ccaaatatgg tcccccatgc 240

ccaccctgcc cagcacctga ggccgccggg ggaccatcag tcttcctgtt ccccccaaaa 300

cccaaggaca ctctcatgat ctcccggacc cctgaggtca cgtgcgtggt ggtggacgtg 360

agccaggaag accccgaggt ccagttcaac tggtacgtgg atggcgtgga ggtgcataat 420

gccaagacaa agccgcggga ggagcagttc aacagcacgt accgtgtggt cagcgtcctc 480

accgtcctgc accaggactg gctgaacggc aaggagtaca agtgcaaggt ctccaacaaa 540

ggcctcccgt cctccatcga gaaaaccatc tccaaagcca aagggcagcc ccgagagcca 600

caggtgtaca ccctgccccc atcccaggag gagatgacca agaaccaggt cagcctgacc 660

tgcctggtca aaggcttcta ccccagcgac atcgccgtgg agtgggaaag caatgggcag 720

ccggagaaca actacaagac cacgcctccc gtgctggact ccgacggctc cttcttcctc 780

tacagcaggc taaccgtgga caagagcagg tggcaggagg ggaatgtctt ctcatgctcc 840

gtgatgcatg aggctctgca caaccactac acacagaaga gcctctccct gtctctgggt 900

<210>  20
<211>  900
<212>  DNA
<213>  Artificial sequence

<220>
<223> Recombinant protein L1-self-mu

<400>  20
cacggcgagg gcaccttcac ctccgacgtg tcctcctatc tcgaggagca ggccgccaag      60

gaattcatcg cctggctggt gcgcggcgcc ggcggtggtg gtggctccgg aggcggcggc      120

tctggtggcg gtggcagcgc tgagtccaaa tatggtcccc catgcccacc ctgcccagca      180

cctgaggccg ccgggggacc atcagtcttc ctgttccccc caaaacccaa ggacactctc      240

atgatctccc ggacccctga ggtcacgtgc gtggtggtgg acgtgagcca ggaagacccc      300

gaggtccagt tcaactggta cgtggatggc gtggaggtgc ataatgccaa gacaaagccg      360

cgggaggagc agttcaacag cacgtaccgt gtggtcagcg tcctcaccgt cctgcaccag      420

gactggctga acggcaagga gtacaagtgc aaggtctcca acaaaggcct cccgtcctcc      480

atcgagaaaa ccatctccaa agccaaaggg cagccccgag agccacaggt gtacaccctg      540

cccccatccc aggaggagat gaccaagaac caggtcagcc tgacctgcct ggtcaaaggc      600

ttctacccca gcgacatcgc cgtggagtgg gaaagcaatg ggcagccgga gaacaactac      660

aagaccacgc ctcccgtgct ggactccgac ggctccttct tcctctacag caggctaacc      720

gtggacaaga gcaggtggca ggaggggaat gtcttctcat gctccgtgat gcatgaggct      780

ctgcacaacc actacacaca gaagagcctc tccctgtctc tgggtggtgg cggtggcggc      840

aactacacct gcgaggtgac cgagctgacc cgcgagggcg agaccatcat cgagctgaag      900


<210>  21
<211>  323
<212>  PRT
<213>  Homo sapiens

<400>  21

Met Trp Pro Leu Val Ala Ala Leu Leu Leu Gly Ser Ala Cys Cys Gly
1               5                   10                  15


Ser Ala Gln Leu Leu Phe Asn Lys Thr Lys Ser Val Glu Phe Thr Phe
            20                  25                  30


Cys Asn Asp Thr Val Val Ile Pro Cys Phe Val Thr Asn Met Glu Ala
        35                  40                  45


Gln Asn Thr Thr Glu Val Tyr Val Lys Trp Lys Phe Lys Gly Arg Asp
    50                  55                  60

Ile Tyr Thr Phe Asp Gly Ala Leu Asn Lys Ser Thr Val Pro Thr Asp
65                  70                  75                  80

Phe Ser Ser Ala Lys Ile Glu Val Ser Gln Leu Leu Lys Gly Asp Ala
                85                  90                  95

Ser Leu Lys Met Asp Lys Ser Asp Ala Val Ser His Thr Gly Asn Tyr
            100                 105                 110

Thr Cys Glu Val Thr Glu Leu Thr Arg Glu Gly Glu Thr Ile Ile Glu
        115                 120                 125

Leu Lys Tyr Arg Val Val Ser Trp Phe Ser Pro Asn Glu Asn Ile Leu
    130                 135                 140

Ile Val Ile Phe Pro Ile Phe Ala Ile Leu Leu Phe Trp Gly Gln Phe
145                 150                 155                 160

Gly Ile Lys Thr Leu Lys Tyr Arg Ser Gly Gly Met Asp Glu Lys Thr
                165                 170                 175

Ile Ala Leu Leu Val Ala Gly Leu Val Ile Thr Val Ile Val Ile Val
            180                 185                 190

Gly Ala Ile Leu Phe Val Pro Gly Glu Tyr Ser Leu Lys Asn Ala Thr
        195                 200                 205

Gly Leu Gly Leu Ile Val Thr Ser Thr Gly Ile Leu Ile Leu Leu His
    210                 215                 220

Tyr Tyr Val Phe Ser Thr Ala Ile Gly Leu Thr Ser Phe Val Ile Ala
225                 230                 235                 240

Ile Leu Val Ile Gln Val Ile Ala Tyr Ile Leu Ala Val Val Gly Leu
                245                 250                 255

Ser Leu Cys Ile Ala Ala Cys Ile Pro Met His Gly Pro Leu Leu Ile
            260                 265                 270

Ser Gly Leu Ser Ile Leu Ala Leu Ala Gln Leu Leu Gly Leu Val Tyr
        275                 280                 285

Met Lys Phe Val Ala Ser Asn Gln Lys Thr Ile Gln Pro Pro Arg Lys
    290                 295                 300

Ala Val Glu Glu Pro Leu Asn Ala Phe Lys Glu Ser Lys Gly Met Met
305                 310                 315                 320

Asn Asp Glu